# EUROPEAN PATENT SPECIFICATION

(11) **EP 0 793 723 B1**
(45) Date of publication and mention of the grant of the patent: **12.10.2005**
(21) Application number: 96900577.6
(22) Date of filing: 03.01.1996
(51) Int. Cl.: C12N 15/31, C07K 14/31, C07K 16/12, A61K 38/16

(54) **NEW STAPHYLOKINASE DERIVATIVES**
NEUE STAPHYLOKINASE DERIVATE
NOUVEAUX DERIVES DE STAPHYLOKINASE

(30) Priority: 06.01.1995 EP 95200023; 11.01.1995 US 371505; 09.06.1995 EP 95201531; 06.07.1995 US 499092; 17.11.1995 JP 29978195
(43) Date of publication of application: 10.09.1997
(73) Proprietor: Leuven Research & Development V.Z.W., B-3000 Leuven (BE); Collen, Désiré José, B-3020 Winksele-Herent (BE)
(72) Inventor: COLLEN, Desiré José, 3020 Winksele-Herent (BE)
(74) Representative: Van Someren, Petronella F. H. M.
(86) International application number: PCT/EP1996/000081
(87) International publication number: WO 1996/021016

(56) References cited:
- WO-A-93/13209
- DD-A- 245 444
- EUROPEAN JOURNAL OF BIOCHEMISTRY, vol. 223, no. 1, July 1994, pages 303-308, XP002001163 GASE A. ET AL.: "The thermostability of natural variants of bacterial plasminogen-activator staphylokinase"

## Description

This invention relates to new staphylokinase derivatives with reduced immunogenicity, and their use in the treatment of arterial thrombosis and for the preparation of a pharmaceutical composition for treating arterial thrombosis. More in particular it relates to the use of engineered staphylokinase derivatives for the preparation of a pharmaceutical composition for treating myocardial infarction.

Thrombotic complications of cardiovascular diseases are a main cause of death and disability and, consequently, thrombolysis (i.e. pharmacological dissolution of the blood clot) could favorably influence the outcome of such life-threatening diseases as myocardial infarction, cerebrovascular thrombosis and venous thromboembolism. Thrombolytic agents are plasminogen activators that convert plasminogen, the inactive proenzyme of the fibrinolytic system in blood, to the proteolytic enzyme plasmin. Plasmin dissolves the fibrin of a blood clot, but may also degrade normal components of the hemostatic system and induce the so-called "lytic state". Physiological fibrinolysis however is fibrin-oriented as a result of specific molecular interactions between tissue-type plasminogen activator, fibrin, plasmin(ogen) and α₂-antiplasmin (1,2).

Currently, six thrombolytic agents are either approved for clinical use or under clinical investigation in patients with acute myocardial infarction. These include streptokinase, urokinase, recombinant tissue-type plasminogen activator (rt-PA) or derivatives of it, anisoylated plasminogen streptokinase activator complex (APSAC), recombinant single chain urokinase-type plasminogen activator (rscu-PA, recombinant prourokinase), and recombinant staphylokinase (Sak) (2,3). In patients with acute myocardial infarction, reduction of infarct size, preservation of ventricular function and reduction in mortality has been observed following treatment with either streptokinase, rt-PA or APSAC (2).

One of the thrombolytic agents currently routinely used in therapy is streptokinase, a Mᵣ 45,000 protein secreted by β-hemolytic streptococci. Its administration is however associated with extensive systemic fibrinogen breakdown and its efficacy for coronary thrombolysis in patients with evolving acute myocardial infarction is limited, amounting to approximately 50 percent coronary artery recanalization within 90 minutes (2). Furthermore, exposure to streptokinase provokes allergic reactions in about 5 percent of treated patients and consistently induces specific antibody formation which precludes its repeated use within months or years (4).

Staphylokinase, a protein produced by certain strains of Staphylococcus aureus, which was shown to have profibrinolytic properties more than 4 decades ago (5-7), also appears to constitute a potent thrombolytic agent in patients with acute myocardial infarction (8). The staphylokinase gene has been cloned from the bacteriophages sakφC (9) and sak42D (10) as well as from the genomic DNA (sakSTAR) of a lysogenic Staphylococcus aureus strain (11). It has been expressed under the control of the λPR promoter and its own translation signals in Escherichia coli and also under the control of its natural promoter and translation signals in Bacillus subtilis or Escherichia coli, resulting in accumulation of the gene product in the periplasmic space or in the culture medium, respectively (10-13).

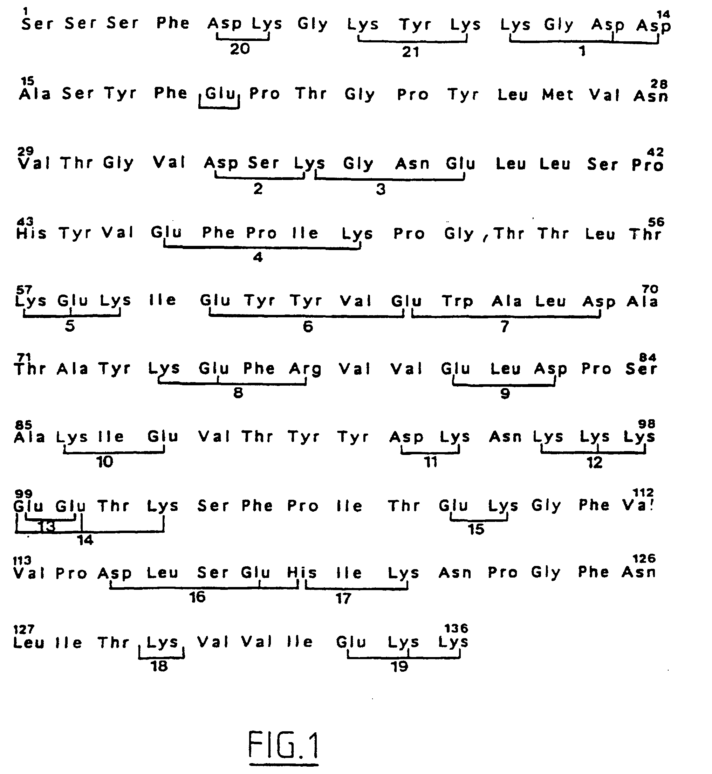

The staphylokinase gene encodes a protein of 163 amino acids, with amino acid 28 corresponding to the NH₂-terminal residue of full length mature staphylokinase (10,14,15). The protein sequence of the wild-type variant SakSTAR (15) is represented in Figure 1. Only four nucleotide differences were found in the coding regions of the sakφC, sak42D and sakSTAR genes, one of which constituted a silent mutation (10,14,15).

Several molecular forms of staphylokinase have been purified with slightly different Mᵣ (16,500 to 18,000 on SDS-PAGE) and iso-electric points (11-13). Lower Mᵣ derivatives of mature staphylokinase were obtained lacking the 6 (Sak-Δ6) or the 10 (Sak-Δ10) NH₂-terminal amino acids. Upon interaction with plasmin(ogen) in a buffer milieu, mature staphylokinase (NH₂-terminal Ser-Ser-Ser) is rapidly and quantitatively converted to Sak-Δ10 (NH₂-terminal Lys-Gly-Asp-). Mature staphylokinase and Sak-Δ10 were shown to have the same fibrinolytic activity (11,12).

The amino acid in position 26 appears to be of crucial importance for the activation of plasminogen by staphylokinase. Indeed, substitution of the unique Met residue in position 26 with either Arg or Val results in loss of the functional activity, whereas substitution with Leu or Cys has little or no effect on the activity (16). Because none of the single amino acid exchanges causes significant changes of the solution structure of the mutant proteins the mechanism of this differential behavior remains enigmatic.

In a plasma milieu, staphylokinase is able to dissolve fibrin clots without associated fibrinogen degradation (17-19). This fibrin-specificity of staphylokinase is the result of reduced inhibition by α₂-antiplasmin of plasmin.staphylokinase complex bound to fibrin, recycling of staphylokinase from the plasmin.staphylokinase complex following inhibition by α₂-antiplasmin, and prevention of the conversion of circulating plasminogen.staphylokinase to plasmin.staphylokinase by α₂-antiplasmin (20-22). In several experimental animal models, staphylokinase appears to be equipotent to streptokinase for the dissolution of whole blood or plasma clots, but significantly more potent for the dissolution of platelet-rich or retracted thrombi (23,24).

The encouraging results obtained with staphylokinase in animal models of thrombosis, have formed the basis for its evaluation, on a pilot scale, in patients with acute myocardial infarction (3,25). In 4 of 5 patients with acute myocardial infarction 10 mg recombinant staphylokinase (SakSTAR), given intravenously over 30 min, was found to induce angiographically documented coronary artery recanalization within 40 minutes. Plasma fibrinogen and α₂-antiplasmin levels were unaffected (residual levels at 40 min of 90-95% of baseline) and allergic reactions were not observed (3). In a second series of 5 patients with acute coronary occlusion, intravenous administration of 10 mg staphylokinase (SakSTAR) over 30 min induced recanalization in all patients within 20 min, without associated fibrinogen degradation (25). Control angiography at 24 hours showed that recanalization persisted.

The immunogenicity of staphylokinase (SakSTAR) as compared to streptokinase was studied in dogs (23) and baboons (24). In aggregate, these experimental animal data suggested a lower immunogenicity of staphylokinase as compared to streptokinase. However, in the first 5 patients with acute myocardial infarction given an intravenous infusion of 10 mg staphylokinase over 30 min, neutralizing antibody titers against staphylokinase (SakSTAR) were low at baseline and up to 6 days after infusion, but high titers (staphylokinase neutralizing titers of 12-42 µg/ml plasma) of antibodies were consistently demonstrable in plasma at 14-35 days (3). These observations were fully confirmed in the second pilot trial in 5 patients (25). Thus with respect to immunogenicity, the initial observations in man were not as encouraging as the experience in experimental animals. Thus, like streptokinase, staphylokinase administration would be restricted to single use. However, the absence of cross-reactivity of induced antibodies against staphylokinase and streptokinase (26,27) suggests that the administration of both substances would not be mutually exclusive.

The intrinsic immunogenicity of streptokinase and staphylokinase clearly hampers their unrestricted use. Not only will patients with preexisting high antibody titers be refractory to the thrombolytic effect of these agents, but allergic side effects and occasional life-threatening anaphylaxis may occur (28). Because both streptokinase and staphylokinase are heterologous proteins, it is not obvious that their immunogenicity could be reduced by protein engineering. Indeed, no successful attempts to generate active low molecular weight fragments from streptokinase have been reported. In staphylokinase, deletion of the NH₂-terminal 17 amino acids or the COOH-terminal 2 amino acids inactivates the molecule, which in addition is very sensitive to inactivation by site-specific mutagenesis (25,29).

Nevertheless, we have, surprisingly, found that the wild-type staphylokinase variant SakSTAR (8,15) contains three non-overlapping immunodominant epitopes, at least two of which can be eliminated by specific site-directed mutagenesis, without inactivation of the molecule. These engineered staphylokinase variants are less reactive with antibodies elicited in patients treated with wild-type staphylokinase, and are significantly less immunogenic than wild-type staphylokinase, as demonstrated in rabbit and baboon models and in patients with peripheral arterial occlusion.

The present invention thus relates to staphylokinase derivatives showing a reduced immunogenicity as compared to wild-type staphylokinase. having essentially the amino acid sequence as depicted in figure 1 in which one or more amino acids in one or more underlined clusters have been replaced by another amino acid thus altering the corresponding epitope(s). Preferably the amino acids are replaced by alanine. The derivatives have essentially the amino acid sequence of wild-type staphylokinase or modified versions thereof, but at least one immunodominant epitope is eliminated without destroying the biological activity of the derivatives. By altering the epitope(s) the reactivity of the derivatives with a monoclonal antibody panel directed to one or more of three epitope clusters I, II and III is reduced. This indicates that by replacing the wild-type amino acids with alanine the antigenicity of staphylokinase is reduced.

The invention in particular relates to staphylokinase derivative M8 having the amino acid sequence as depicted in figure 1 in which the amino acids Lys on position 74, Glu on position 75 and Arg on position 77 in the underlined cluster 8 have been replaced by alanine thus altering the corresponding epitope, to staphylokinase derivative M3 having the amino acid sequence as depicted in figure 1 in which the amino acids Lys on position 35 and Glu on position 38 in the underlined cluster 3 have been replaced by alanine thus altering the corresponding epitope, to staphylokinase derivative M9 having the amino acid sequence as depicted in figure 1 in which the amino acids Glu on position 80 and Asp on position 82 in the underlined cluster 9 have been replaced by alanine thus altering the corresponding epitope, to staphylokinase derivative M3.8 having the amino acid sequence as depicted in figure 1 in which the amino acids Lys on position 35, Glu on position 38, Lys on position 74, Glu on position 75 and Arg on position 77 in the underlined clusters 3 and 8 have been replaced by alanine thus altering the corresponding epitopes and to staphylokinase derivative M8.9 having the amino acid sequence as depicted in figure 1 in which the amino acids Lys on position 74, Glu on position 75, Arg on position 77, Glu on position 80 and Asp on position 82 in the underlined clusters 8 and 9 have been replaced by alanine thus altering the corresponding epitopes. Thus M3.8 and M8.9 are double mutants having two epitopes destroyed.

The invention demonstrates that engineered variants of staphylokinase with reduced immunogenicity can be practical alternative thrombolytic agents to streptokinase or wild-type staphylokinase.

The invention also relates to a method for producing the derivatives of the invention by preparing a DNA fragment comprising at least the part of the coding sequence of staphylokinase that provides for its biological activity; performing in vitro site-directed mutagenesis on the DNA fragment to replace one or more codons for wild-type amino acids by a codon for another amino acid; cloning the mutated DNA fragment in a suitable vector; transforming or transfecting a suitable host cell with the vector; and culturing the host cell under conditions suitable for expressing the DNA fragment. Preferably the DNA fragment is a 453 bp EcoRI-HindIII fragment of the plasmid pMEX602SAK, the in vitro site-directed mutagenesis is performed by an oligonucleotide-directed mutagenesis system using the plasmid pMa/c and the repair deficient E. coli strain WK6MutS, and the mutated DNA fragment is cloned in E. coli strain WK6.

The invention also relates to pharmaceutical compositions comprising at least one of the staphylokinase derivatives according to the invention together with a suitable excipient, for treatment of arterial thrombosis. Pharmaceutical compositions, containing less immunogenic staphylokinase variants as the active ingredient, for treating arterial thrombosis in human or veterinary practice may take the form of powders or solutions and may be used for intravenous or intraarterial administration. Such compositions may be prepared by combining (e.g. mixing, dissolving etc.) the active compound with pharmaceutically acceptable excipients of neutral character (such as aqueous or nonaqueous solvents, stabilizers, emulsifiers, detergents, additives), and further, if necessary with dyes. The concentration of the active ingredient in a therapeutical composition may vary widely between 0,1% and 100%, dependent on the character of the disease and the mode of administration. Further the dose of the active ingredient to be administered may vary between 0,05 mg and 1,0 mg per kg of body weight.

Furthermore the invention relates to the use of the staphylokinase derivatives for the treatment of arterial thrombosis, in particular myocardial infarction, and to the use of staphylokinase derivatives for the preparation of a pharmaceutical composition for the treatment of arterial thrombosis, in particular myocardial infarction.

In the above and the following the terms "derivatives", "mutants" and "variants" are used interchangeably.

The present invention will be demonstrated in more detail in the following examples, that are however not intended to be limiting to the scope of the invention. Based on the present invention several variants and improvements will be obvious for the person skilled in the art. Thus random mutagenesis starting from the combination mutant 3.8 and from the combination mutant 8.9 is likely to generate alternative mutants with reduced immunogenicity and possibly increased function activity, whereas alternative mutagenesis in the epitope neutralizing clusters will yield other variants with reduced immunogenicity.

### EXAMPLE 1

### Epitope mapping of wild-type staphylokinase

The epitope specificity of a panel of 17 murine monoclonal antibodies raised against wild-type staphylokinase (SakSTAR variant) was determined by real-time biospecific interaction analysis (BIA) using the BIAcore™ instrument (Pharmacia, Biosensor AB, Uppsala, Sweden). Monoclonal antibodies against SakSTAR were produced essentially by the method of Galfré and Milstein (30). BALB/c mice were immunized by subcutaneous injection of 10 µg SakSTAR in complete Freund's adjuvant, which was followed 2 weeks later by intraperitoneal injection of 10 µg SakSTAR in incomplete Freund's adjuvant. After an interval of at least 6 weeks, the mice were boosted intraperitoneally with 10 µg SakSTAR in saline on days 4 and 2 before the cell fusion. Spleen cells were isolated and fused with P3X63-Ag.8-6.5.3 myeloma cells (obtained from Dr. O. Schönherr, Organon, Oss, The Netherlands) according to Fazekas de St. Groth and Scheidegger (31). After selection in hypoxanthine, aminopterin, thymidine medium, the supernatants were screened for specific antibody production with a one-site noncompetitive micro-ELISA using microtiter plates coated with staphylokinase. The bound immunoglobulins were detected with horseradish peroxidase (HP)-conjugated rabbit anti-mouse IgG (32). Positive clones were used for the production of ascitic fluid in pristane-primed BALB/c mice (33). The IgG fraction of the monoclonal antibodies was purified from ascites by affinity chromatography on Protein A-Sepharose (34).

This biospecific interaction analysis technique, based on surface plasmon resonance (SPR) allows direct measurement of interactions in real time without the use of labels (35). Staphylokinase (SakSTAR) was immobilized on the surface of Sensor Chip CM5 using the Amine Coupling kit (Pharmacia Biosensor AB), as recommended by the manufacturer. This procedure links primary amino groups in the ligand to the carboxymethylated dextran surface of the Sensor Chip (36). Immobilization was performed from protein solutions at a concentration of 10 µg/ml in 10 mM Na-acetate at pH 5.0, at a flow of 5 µl/min during 6 min. This resulted in covalent attachment of 1,000-1,500 RU (resonance units) of staphylokinase moieties (corresponding to approximately 0.07 pmole/mm²) (37). The second interacting component (the analyte: i.e. monoclonal antibody) was injected in solution over the sensor. The concentration of free analyte was kept constant through a continuous flow of solution at 20°C past the sensor surface. At least four concentrations of each analyte (range 0-400 nM or 0-50 µM) in 10 mM HEPES, 3.4 mM EDTA, 0.15 M NaCl and 0.005% Surfactant P20, pH 7.2, were injected at a flow rate of 5 µl/min during 6 min in the association phase. Then sample was replaced by buffer, also at a flow rate of 5 µl/min during 6 to 30 min. After each cycle, the surface of the sensor chip was regenerated by injection of 5 µl of 15 mM HCl. Association (kₐₛₛ) and dissociation (k_{diss}) rate constants were derived from the sensorgrams as described in detail elsewhere (38). The equilibrium association constants (K_{A}), calculated as the ratio of kₐₛₛ and k_{diss}, for the binding to wild-type staphylokinase of the panel of 17 monoclonal antibodies studied, ranged between 0.6 and > 25 x 10⁹ M⁻¹ (median value 10¹⁰ M⁻¹) (Table 1).

In table 1 the column indicated with "ID" states the various staphylokinase derivatives. Indications "17G11", "26A2" etc. refer to monoclonal antibodies binding to the indicated epitope clusters I, II and III. In the column "variant" the mutated amino acids and their position are indicated in the one letter code for amino acids. Epitope cluster I is recognized by the antibodies 17G11, 26A2, 30A2, 2B12 and 3G10, whereas epitope cluster II is recognized by the antibodies 29C1, 18F12, 14H5, 28H4, 20D6, 32B2 and 7F10, and epitope cluster III by the antibodies 7H11, 25E1, 40C8, 24C4 and 1A10.

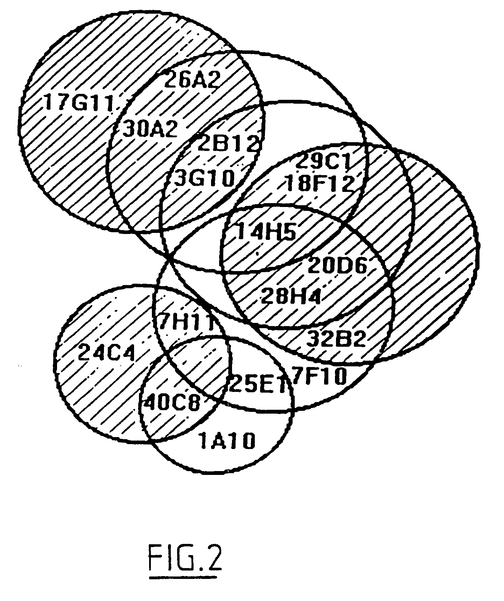

Monoclonal antibodies directed against separate epitopes will bind independently of each other, whereas monoclonal antibodies directed against closely related epitopes will interfere with each other's binding. Therefore, the epitope specificity of a panel monoclonal antibodies is most easily determined by testing the ability of pairs of monoclonal antibodies to bind simultaneously to the antigen. Real-time biospecific interaction analysis (BIA) can be used to measure competitive binding of pairs of monoclonal antibodies to staphylokinase linked to the sensor chip surface. The analysis was carried out as described in Application Note 101 (Pharmacia Biosensor AB). Pair-wise binding tests divided the 17 monoclonal antibodies into 3 groups representing 3 non-overlapping epitopes on the antigen, as illustrated in Figure 2. The independence of these epitopes was confirmed by the direct demonstration of additive binding of the monoclonal antibodies 26A2, 28H4 and 24C4. The antibodies were aligned according to their epitope specificity as illustrated in Table 1.

### EXAMPLE 2

### Construction and epitope mapping of "charged-cluster-to-alanine" variants of staphylokinase

In the "charged-cluster-to-alanine" scan, clusters of hydrophilic charged amino acids were targeted. Staphylokinase (SakSTAR) contains 45 charged amino acids (2 His, 14 Glu, 8 Asp, 1 Arg and 20 Lys). These charged residues were mutagenized to Ala in clusters of two or three amino acids, as summarized in Figure 1. A total of 21 mutants in which the underlined charged amino acids were replaced by alanine were designed. The amino acids that are to be replaced by alanine are indicated with a small vertical line within the cluster.

Mutants were prepared by site-directed mutagenesis and expressed in E. coli as detailed below. Restriction enzymes were purchased from Pharmacia, Uppsala, Sweden or Boehringer Mannheim (Mannheim, Germany). T4 DNA ligase, Klenow Fragment of E. coli DNA polymerase I and alkaline phosphatase were obtained from Boehringer Mannheim. The oligonucleotide-directed mutagenesis system and the pMa/c plasmids were kindly provided by Corvas (Ghent, Belgium) (39). The expression vector pMEX602SakB was kindly provided by the Institut für Molekulare Biotechnologie, Jena, Germany (25). M123KO7 helper phage was purchased from Promega (Leiden, The Netherlands). Luria Broth growth medium was purchased from Life Technologies (Merelbeke, Belgium). Plasminogen was purified from human plasma as described elsewhere (40).

Enzyme reactions were performed using the conditions suggested by the suppliers. Plasmid DNA was isolated using a QIAGEN-purification protocol (provided by Westburg, Leusden, The Netherlands). Transformations of E. coli were performed utilizing the calcium phosphate procedure. DNA sequencing was performed using the dideoxy chain termination reaction method and the Automated Laser fluorescent A.L.F.™ (Pharmacia). Site directed mutagenesis for the mutants D5,K6 (M20) until K86,E88 (M10), was performed using the pMa/c, using the repair deficient E. coli strain WK6MutS. Propagation of the plasmids pMa/c or derivatives, preparation of single stranded DNA and expression was done in E. coli WK6 (39). The mutants D93,K94 (M11) until K134,K135,K136 (M19) were constructed in the Institut für Molekulare Biotechnologie Jena, Germany as previously described (16). The chromogenic substrate (S2403) L-Pyroglutamyl-L-phenylalanyl-L-lysine-P-nitroanaline hydrochloride was purchased from Chromogenix. ¹²⁵I-labeled fibrinogen was purchased from Amersham.

The 453-base pair EcoRI-HindIII fragment containing the entire coding region for SakSTAR was cut out of the plasmid pMEX602SakB (ampicillin resistant) and cloned into the EcoRI-HindIII sites of the pMc5-8 plasmid (chloramphenicol resistant) yielding pMc-STAR. For in vitro site-directed mutagenesis, single stranded DNA of this construct was prepared by transformation of the pMc-STAR construct in E. coli and injection of an overnight culture with helper phage M13K07. Four hours after injection, cells were isolated from the medium by PEG-precipitation and phenol-chloroform extraction. Subsequently, single stranded pMc-STAR was hybridized with single stranded pMa (EcoRI-HindIII) vector DNA and the appropriate 28 to 44 base synthetic oligonucleotide with a silent mutation creating or deleting a restriction site. Extension reactions were carried out with the Klenow fragment of DNA polymerase as described. After transformation of E. coli WK6MutS and selection on ampicillin, colonies were grown on nitrocellulose membranes, denatured in situ and DNA was hybridized overnight at room temperature using the respective radiolabeled mutant oligonucleotides (1.5 x 10⁸ cpm of [γ³²P]-ATP used for T4 polynucleotide kinase labeling of 20-30 ng of oligonucleotide). Filters were washed at 42°C using solutions containing 0.1% SDS and 2x SSC, 1x SSC, 0.2x SSC, 0.1x SSC. Plasmid DNA was extracted from 10 ml bacterial cultures form each positive clone and analyzed by restriction enzyme digestion. The desired mutations were confirmed by sequencing of the complete coding sequence using A.L.F.™.

The mutated HindIII-EcoRI fragment was then ligated back into the pMEX602SakB expression vector containing the pTaq promoter (39). The mutant proteins were produced intracellularly and in soluble form in E. coli WK6 cells transformed with this vector. The mutants were purified from the sonicated bacterial extracts using cation exchange and hydrophobic interaction chromatography (25).

SakSTAR mutants were obtained with yields ranging between 10 and 80 mg/l, representing recoveries of 15 to 88% of the starting material. The purified material was pure as shown by electrophoresis on non reduced 10-15% gradient gels (not shown). NH₂-terminal amino acid analysis confirmed the Ser-Ser-Ser-Phe-Asp sequence of mature staphylokinase. A more detailed biochemical characterization of these staphylokinase mutants has been reported elsewhere (41).

Protein concentrations were determined according to Bradford (42). The fibrinolytic activities of SakSTAR solutions were determined with a chromogenic substrate assay carried out in microtiters plates using a mixture of 80 µl SakSTAR solution and 100 µl Glu-plasminogen solution (final concentration 0.5 mM). After incubation for 30 min at 37°C, generated plasmin was quantitated by addition of 30 µl S2403 (final concentration 1 µM) and measurement of the absorption at 405 nm. The activity was expressed in home units (HU) by comparison with an in-house standard (lot STAN5) which was assigned an activity of 100,000 HU per mg protein as determined by amino acid composition (11). SDS-PAGE was performed with the Phast System™ (Pharmacia, Uppsala, Sweden) using 10-15% gradient gels and Coomassie Brilliant blue staining. Reduction of the samples was performed by heating at 100°C for 3 min in the presence of 1% SDS and 1% dithioerythritol.

The construction, production and purification of mutants M3.8 and M8.9 were carried out as described in detail below. The oligonucleotides, used for the construction, 5'-ATAGCAATGCATTTCCTGCACTATCAAC-3' (M3), 5'-CTAATTCAACTACTGCAAACGCTGCATATGCTGTCGCATC-3' (M8), and 5'-TTTGCGCTTGGCGCCAATGCAACTACTCTAAACTCTTTATAT-3' (M9) were custom synthesized by Pharmacia Biotech.

For the construction of mutant M3.8, single-stranded pMc-STAR and the EcoRI-HindIII fragment of pMa5-8 were used to prepare a gapped-duplex DNA molecule, which was hybridized with the 28 base synthetic oligonucleotide M3 (containing a NsiI restriction site). Extension reactions were carried out with the Klenow fragment of DNA polymerase and ligase as described. After transmission of E. coli WK6MutS and selection on ampicillin, 81 colonies were grown on nitrocellulose membranes, denatured in situ and DNA was hybridized overnight at room temperature using radiolabeled M3 oligonucleotide (1.5 x 10⁸ cpm of [γ³²P]-ATP for T4 polynucleotide kinase labeling of 20-30 ng of oligonucleotide). Filters were washed at 42°C using solutions containing 0.1% SDS and 2 x SSC, 1 x SSC, 0.2 x SSC, 0.1 x SSC. DNA of 1 selected clone out of 16 positives was prepared from 150 ml bacterial cultures and analyzed by restriction enzyme digestion with NsiI and PvuI. The M3 mutation (pMa-STAR3) was confirmed by sequencing of the complete coding region using A.L.F.™ Then single stranded DNA was prepared by transformation of pMa-STAR3 in E. Coli as described above. Single stranded pMa-STAR3 was hybridized with pMc (EcoRI-HindIII) and with the 40 base synthetic oligonucleotide M8 which contains a NdeI restriction site, as described above. After transformation of E. coli WK6MutS and selection on chloramphenicol, 100 colonies were grown on nitrocellulose membranes and hybridized with labeled M8 oligonucleotide. Positive clones (2 out of 7) were grown, and analyzed by restriction enzyme digestion (NsiI-HindIII and NdeI) yielding one positive clone. The double mutant M3.8 was then ligated back into the pMEX602SakB expression vector. Out of 12 minipreparations of DNA, 6 had the correct restriction pattern. One of these clones (pMEXSakSTAR.M38) was sequenced and used for preparation of mutant M3.8 under control of the IPTG inducible tac promoter and two Shine-Dalgarno sequences in tandem.

100 µl of a suspension of E. coli WK6 cells transformed with the recombinant plasmid pMEXSakSTAR.M38 were incubated in 100 ml LB medium (Gibco/BRL) containing 100 µg/ml of ampicillin. The mixture was incubated overnight at 37°C while shaking at 200 rpm, resulting in a cell density of approximately 5 absorbance units at 600 nm. Aliquots of 20 ml were transferred to 2 1 volumes (in 5 1 flasks) of LB medium containing 100 µg/ml ampicillin. The mixtures were incubated for 3 hours at 37°C while shaking before addition of 200 µM IPTG for induction of M3.8 expression, which was allowed to proceed for 4 hours. The cells were pelleted by centrifugation at 4,000 rpm for 20 min, resuspended in 1/10 volume of 0.01 M phosphate buffer, pH 6.5, and disrupted by sonication at 0°C. Cell debris was removed by centrifugation for 30 min at 20,000 rpm and the supernatant was stored at -20°C until used.

Pooled cleared cell lysates (2 liter volumes) from 20 to 30 liter bacterial cultures were pH adjusted to 5.9, sterilized by filtration through a 0.22 µm Sartorius filter and applied to a 5x25 cm column of SP-Sepharose, preconditioned with 0.5 M NaOH and with sterilized 0.01 M phosphate buffer, at a flow rate of 12 ml/min and at 4°C in a laminar flow. The column was washed with 2 to 3 liter buffer and eluted with a salt gradient from 0 to 1 M over 500 ml and from 1 M to 2 M over 250 ml at a flow rate of 10 ml/min and at 4°C. The M3.8 containing fractions, localized by SDS gel electrophoresis, were pooled (approximately 200 ml) and dialyzed against 15 liter sterilized 0.01 M phosphate buffer, pH 8.0, at 4°C. The dialyzed material was centrifuged at 4,000 rpm for 30 min, sterilized again by filtration and applied to a 2.5 x 12 cm column of Q-Sepharose fast flow, preconditioned with 0.5 M NaOH and with sterilized 0.01 M phosphate buffer, pH 8.0, at a flow rate of 3 ml/min at 4°C. The column was washed with approximately 600 ml 0.01 M phosphate buffer, pH 8.0, at a flow rate of 8 ml/min and eluted with a salt gradient from 0 to 0.17 M over 30 ml, from 0.17 to 0.2 M over 100 ml and from 0.2 M to 1.5 M over 200 ml, at a flow rate of 4 ml/min. The M3.8 containing fractions, localized by SDS gel electrophoresis, were pooled, the protein concentration was adjusted to 1 mg/ml and the material was sterilized by filtration through a 0.22 µm Millipore filter. Three preparations of M3.8 yielded 80 ± 25 mg pure protein (mean ± SD) with a specific activity of 45,000 ± 5,200 HU/mg.

For the construction of mutant M8.9, single-stranded pMc-STAR and the EcoRI-HindIII fragment of pMa5-8 were used to prepare a gapped-duplex DNA molecule, which was hybridized with the 42 base synthetic oligonucleotide M9 containing a NarI restriction site. Extension reactions were carried out with the Klenow fragment of DNA polymerase and ligase as described. After transformation of E. coli WK6MutS and selection on ampicillin, colonies were grown on nitrocellulose membranes, denatured in situ and DNA was hybridized overnight at room temperature using radiolabeled M9 oligonucleotide (1.5 x 10⁸ cpm of [γ³²P]-ATP for T4 polynucleotide kinase labeling of 20-30 ng of oligonucleotide). Filters were washed at 42°C using solutions containing 0.1% SDS and 2x SSC, 1x SSC, 0.2x SSC, 0.1x SSC. DNA from 2 selected clones out of 4 positives was prepared and 1 of them (pMA-STAR9) was characterized by nucleotide sequence analysis using A.L.F.™. The EcoRI-HindIII insert from pMa-STAR9 was then ligated back into the pMEX602SakB expression vector. The clones (58) were screened by in situ hybridization with radiolabeled M9 oligonucleotide as a probe. One clone, pMEXSakSTAR.M9, was characterized by nucleotide sequence analysis and subsequently used for the construction of mutant M8.9.

To construct M8.9, mutation 8 was introduced in M9 by polymerase chain reaction (PCR). PCR was performed in a total volume of 100 µl using 5 U enzyme and 1 µg of each of the following primers: oligonucleotide II= 5'-CAGGAAACAGAATTCAGGAG, oligonucleotide III= 5'-TATATAATATTCGACATAGTATTCAATTTTT-3', oligonucleotide IV= 5'-TATCCCGGGCATTAGATGCGACAGCATATGCAGCGTTTGCAGTA-3' and oligonucleotide V= 5'-CAAAACAGCCAAGCTTCATTCATTCAGC-3'. The concentrations of dATP, dCTP, dGTP and dTTP were 200 µM. Denaturation was carried out for 1 min at 94°C, annealing for 2 min at 55°C and extension for 1.5 min at 72°C. After 30 cycli samples were incubated for 10 min at 72°C and cooled to 4°C. In a first PCR reaction 2 ng of pMEXSakSTAR.M9 was amplified using oligonucleotides IV and V as primers. The PCR amplicon was digested with Smal and HindIII and purified after electrophoresis in a 1.5 % agarose gel using a Prep-A-gene kit (Bio-Rad Laboratories, Hercules, CA, USA). The resulting fragment was cloned into the SmaI-HindIII sites of pUC18 (Pharmacia BioTech, Uppsala, Sweden) using the rapid DNA ligation kit (Boehringer Mannheim). After transformation in E. coli WK6 cells, DNA from 12 colonies was prepared and all 12 generated a fragment of approximately 230 base pairs when digested with EcoRI and HindIII. One of these DNA's (pUC18-M89Δ) was used for cloning of a second PCR product (see below). A second PRC reaction was performed on 2 ng of pMEXSakSTAR.M9 using oligonucleotides II and III as primers. The PCR reaction product was digested with SspI and EcoRI and further purified as described above. The resulting fragment was ligated into the SmaI-EcoRI sites of pUC18-M89Δ. After transformation in E. coli WK6 cells, 6 clones were selected for DNA preparation. Five out of 6 generated a fragment of approximately 453-base pairs following digestion with EcoRI and HindIII. This fragment coding for the entire mutant M8.9 was cloned into the EcoRI-HindIII sites of the expression vector pMEX602SakB. After transformation of E. coli WK6 cells, DNA from 6 colonies was analyzed by digestion with EcoRI and HindIII generating a fragment of approximately 453-base pairs in all cases. One of these DNA was further characterized by nucleotide sequence analysis.

100 ml of LB medium (GIBCO/BRL) containing 100 µg/ml ampicillin was inoculated with 100 µl of a suspension of E. coli WK6 cells transformed with the recombinant plasmid pMEXSakSTAR.M89. The culture was incubated overnight at 37°C while shaking at 140 rpm to a cell density of approximately 5 absorbance units of 600 nm. Aliquots of 4 ml were used to inoculate 2 liter cultures (in SL baffled flasks) in "Terrific Broth" medium containing 150 µl/ml ampicillin. The cultures were incubated for about 20 hrs at 30°C and at 140 rpm, resulting in a final cell density of approximately 4.10⁹ cells/ml . The cells were pelleted by centrifugation at 4,000 rpm for 20 min, resuspended in 1/5 volume of 0.01 M phosphate buffer, pH 6.5, and disrupted by sonication at 0°C. The pH was then adjusted to 5.8 and the cells debris were removed by 30 min centrifugation at 20,000 rpm. The supernatant was stored at -20°C until further processed.

Pooled cleared cell lysates (1,800 ml) were pH adjusted to 5.8 and applied to a 2.5 x 20 cm column of SP-Sepharose, preconditioned with 0.5 M NaOH and fresh 0.01 M phosphate, 2.5 M NaCl buffer, pH 7.5, at a flow rate of 2 ml/min at 4°C. The column was washed with 500 µl buffer and eluted with a salt gradient from 0 to 1 M over 200 ml at a flow rate of 6 ml/min.

The pooled M8.9 fractions, identified with SDS gel electrophoresis, were adjusted to 2.5 M with solid NaCl and subjected to hydrophobic interaction chromatography on a 2.5 x 20 cm column of phenyl-Sepharose, preconditioned with 0.5 M NaOH and fresh 0.01 M phosphate, 2.5 M NaCl buffer pH 7.5, at a flow rate of 2 ml/min and 4°C. The column was washed with approximately 500 ml buffer and eluted with 0.01 M phosphate buffer, pH 6.5. The M8.9 containing fractions, localized by SDS gel electrophoresis, were pooled and dialyzed against 2 liter 0.01 M phosphate buffer, pH 9.0. The dialyzed material was centrifuged at 4,000 rpm for 30 min and applied to a 1.6 x 5 cm column of Q-Sepharose fast flow, preconditioned with 0.5 M NaOH and with fresh 0.01 M phosphate buffer, pH 9.0, at a flow rate of 2 ml/min and 4°C. The column was washed with approximately 150 ml 0.01 M phosphate buffer, pH 9.0, and eluted with a salt gradient from 0 to 1 M NaCl over 100 ml, at a flow rate of 4 ml/min. The M8.9 containing fractions, localized by SDS gel electrophoresis were pooled, the protein concentration was adjusted to 1 mg/ml and the material was sterilized by filtration through a 0.22 µM Millipore filter. Three preparations of M8.9 yielded 73 ± 17 mg pure protein with a specific acitivity of 51,000 ± 3,500 HU/mg.

The fibrinolytic activities of the different SakSTAR mutants determined with the chromogenic substrate assay are summarized in table 1.

Of the 21 mutants, designed as illustrated in Figure 1, E99,E100 (M13) and E99,E100,E102 (M14), could not be obtained in purified form, whereas K11,D13,D14 (M1), E46,K50 (M4) and E65,D69 (M7) were inactive. Sixteen mutants, summarized in Table 1 were studied in detail, together with wild-type SakSTAR. Of these mutants, D5,K6 (M20), K8,K10 (M21), D33,K35 (M2), K57,E58,K59 (M5), E61,E65 (M6), K86,E88 (M10), D93,K94 (M11), K96,K97,K98 (M12), E108,K109 (M15), D115,E118,H119 (M16), H119,K121 (M17), K130 (M18) and E134,K135,K136 (M19) reacted with the monoclonal antibody panel is a similar way as SakSTAR. However K35,E38 (M3) and E80,D82 (M9) reacted poorly with the antibody cluster 7H11, 25E1, 40C8, whereas K74,E75,R77 (M8) reacted poorly with the cluster 26A2, 30A2, 2B12 and 3G10. Additivity of epitope elimination was established with the mutants K35,E38/K74, E75,R77 (M3.8) and K74,E75,R77/E80,D82 (M8.9) which combined the reduced reactivity with the monoclonal antibodies of both parent molecules.

### EXAMPLE 3

### Adsorption with wild-type and "charged-cluster-to-alanine" staphylokinase variants of antibodies, elicited in patients by treatment with SakSTAR

In order to obtain information on the epitope specificity of induced antibodies elicited in patients with acute myocardial infarction after treatment with SakSTAR, plasma samples from 16 patients were absorbed with a two-fold molar excess (over the staphylokinase neutralizing activity) of single and combined "charged-cluster-to-alanine" mutants for 10 minutes before determination of residual binding to SakSTAR by biospecific interaction analysis. The staphylokinase-neutralizing activity in these samples was determined as follows. Increasing concentrations of wild-type or variant SakSTAR (50 µl volumes containing 0.2 to 1000 µg/ml) were added to a mixture of 300 µl citrated human plasma and 50 µl buffer or test plasma, immediately followed by addition of 100 µl of a mixture containing thrombin (50 NIH units/ml) and CaCl₂ (25 mM). The plasma clot lysis time was measured and plotted against the concentration of SakSTAR moiety. From this curve the concentration of plasminogen activator that produced complete clot lysis in 20 min was determined. The neutralizing activity titer was determined as the difference between the test plasma and buffer values and was expressed in µg per ml test plasma.

The results are summarized in Table 2. Whereas the wild-type SakSTAR absorbed more than 90 percent of the binding antibodies from all samples, incomplete absorption was observed with mutant K35,E38 (M3) in 4 patients, with mutant K74,E75,R77 (M8) in 12 patients and with mutant E80,D82 (M9) in 5 patients. Absorption with the combination mutants K35,E38/K74,E75,R77 (M3.8) and K74,E75,R77/E80,D82 (M8.9) removed less than 90% of the antibodies in 13 patients (median value of 68 and 65 percent respectively for the 16 patients), whereas, as anticipated, a mixture of the parent molecules of the combination mutants (M8 and M3 or M9) consistently absorbed in excess of 90 percent of the antibodies.

### EXAMPLE 4

### Immunogenicity of "charge-cluster to alanine" variants of staphylokinase in rabbits immunized with wild-tyre staphylokinase (SakSTAR), with mutants K35, E3.8 (M3), K74, X75, R77 (M8), and E80, D82 (M9) and with the combination mutants K35,E38/K74, E75, R77 (M3.8) and K74, E75, R77/E80, D82 (M8.9).

The comparative immunogenicity of SakSTAR versus each of the SakSTAR variants, M3, M8, M9, M3.8 and M8.9 was studied following subcutaneous immunization in groups of 4 or 8 rabbits allocated to SakSTAR and in groups of 8 rabbits allocated to the variant. Immunization was carried out by intravenous infusion of 400 µg/kg SakSTAR and of 200 to 1,000 µg/kg of the mutants at week 0 (to determine the baseline clot lysis capacity) followed by subcutaneous injection of 400 µg of the same agent in complete Freund's adjuvant at week 2 and in incomplete Freund's adjuvant at weeks 3 and 5. The immunogenicity was quantitated at 6 weeks by determination of the staphylokinase-neutralizing activity in plasma and the residual thrombolytic potency as detailed below.

Briefly, staphylokinase-neutralizing activity in plasma was determined by adding increasing concentrations of wild-type or mutant SakSTAR (50 µl volumes containing 0.2 to 1000 µg/ml) to a mixture of 300 µl citrated human plasma and 50 µl buffer or rabbit plasma, immediately followed by addition of 100 µl of a mixture containing thrombin (50 NIH units/ml) and CaCl₂ (25 mM). The plasma clot lysis time was measured and plotted against the concentration of SakSTAR or variant. From this curve the concentration of plasminogen activator that produced complete clot lysis in 20 min was determined. The neutralizing activity titer was determined as the difference between the rabbit plasma and buffer values and was expressed in µg per ml rabbit plasma.

The thrombolytic properties were studied using 0.3 ml ¹²⁵I-fibrin labeled platelet-poor rabbit plasma clots, inserted into an extracorporeal arteriovenous loop. An exposed femoral artery was therefore catheterized with a 4 French catheter (Portex White, Portex, Hythe, UK) and connected via two hypodermic syringes to a catheterized ear vein. The blood flow through the extracorporeal loop was maintained at 10 ml/min with a peristaltic pump. ¹²⁵I-fibrin labeled plasma clots were introduced in each of two syringes inserted in the loop. The plasma clots were prepared by mixing 0.3 ml platelet-poor plasma with a trace amount (approximately 1.5 µCi) ¹²⁵I-labeled human fibrinogen solution (Amersham, Buckinghamshire, UK) and 0.07 ml of a mixture of bovine thrombin (15 NIH units/ml) and 0.5 M CaCl₂, followed by incubation for 30 min at 37°C. Thirty min before the start of the infusion, 7.5 mg/kg ridogrel (a combined thromboxane synthase inhibitor and prostaglandin endoperoxide receptor antagonist) (43) was administered as an intravenous bolus to prevent platelet deposition in the extracorporeal loop. The animals were anticoagulated with heparin (300 units/kg followed by a continuous infusion of 200 units/kg/h throughout the experiment) and randomly allocated to infusion with 400 µg/kg SakSTAR, (4 or 8 rabbits) or 200 to 1000 µg/kg SakSTAR variant (8 rabbits). At 6 weeks, half of the rabbits allocated to the SakSTAR variant were again treated with the same SakSTAR variant and the other half with wild type SakSTAR while the rabbits immunized with SakSTAR were either treated with the SakSTAR variant (if this control group consisted of 4 rabbits) or randomized either to wild type SakSTAR or to the SakSTAR variant (if this control group contained 8 rabbits). The thrombolytic agents were given intravenously as a 10% bolus and a 90% infusion over 1 h. The time course of clot lysis was monitored continuously by external gamma counting, using two 3x0.5 inch sodium iodide/thallium crystals (Bicron, Newbury, OH) positioned over the extracorporeal loops. The scintillation crystals were connected to a dedicated Canberra-S100 system (Canberra-Packard, Meriden, CT), and the data were analyzed as described elsewhere (44). At the end of the experiment the residual clots were also recovered from the syringes for determination of their radioisotope content. The animal experiments were conducted conform the guiding principles of the American Physiological Society and the International Committee on Thrombosis and Haemostasis (45).

The immunogenicity of SakSTAR and the respective single mutants (M3, M8 and M9) is compared in Table 3A. Results are expressed as mean ± SD.

In 8 rabbits randomized to mutant M3, the baseline neutralizing activity was 0.0 ± 0.0 µg/ml both against SakSTAR and against M3. Intravenous infusion of 200 µg/kg M3 induced 76 ± 23 per cent clot lysis. These 8 rabbits were then immunized with M3, suspended in 500 µl of complete Freund's adjuvant at week 2 and in 500 µl incomplete Freund's adjuvant at weeks 3 and 5. At week 6, the plasma neutralizing activity was increased to 11 ± 6.7 µg/ml against SakSTAR and to 11 ± 7.2 µg/ml against M3. At week 6, infusion of 400 µg SakSTAR in 4 of these rabbits, selected at random, produced 18 ± 27 per cent clot lysis while infusion of 200 µg/kg M3 in the 4 other rabbits induced 16 ± 17 per cent lysis. In 4 rabbits assigned to SakSTAR, the baseline neutralizing activity was 0.2 ± 0.2 µg/ml against SakSTAR and 0.0 ± 0.0 µg/ml against M3. Intravenous infusion of 400 µg/kg SakSTAR produced 89 ± 8.6 per cent baseline lysis. These 4 rabbits were then immmunized with 400 µg SakSTAR suspended in either 500 µg of complete (at week 2) or incomplete (at weeks 3 and 5) Freund's adjuvant. At week 6 the plasma neutralizing activity was increased to 35 ± 23 µg/ml against SakSTAR and to 19± 13 µg/ml against M3. Intravenous infusion of 200 µg/kg SakSTAR.M3 in these 4 rabbits at week 6 induced 9.3 ± 8.2 per cent lysis.

In 8 rabbits assigned to the mutant M8, the baseline neutralizing activity in plasma was 1.4 ± 0.2 µg/ml against SakSTAR and 0.6 ± 0.5 µg/ml against M8. Intravenous infusion of 1000 µg/kg M8 produced 41 ± 13 per cent lysis. These rabbits were then immunized with 400 µg M8 suspended in complete Freund's adjuvant at week 2 and with the same amount in incomplete Freund's adjuvant at weeks 3 and 5. At week 6 the plasma neutralizing activity was increased to 3.8 ± 1.8 µg/ml against SakSTAR and to 5.9 ± 2.7 µg/ml against M8. Infusion of 400 µg/kg SakSTAR in 4 of these rabbits produced 49 ± 28 per cent clot lysis whereas infusion of 1000 µg/kg M8 in the 4 other rabbits produced 24 ± 11 per cent lysis. In 8 rabbits assigned to the SakSTAR group the baseline neutralizing activity in plasma was 0.9 ± 0.6 µg/ml against SakSTAR and 0.6 ± 0.3 µg/ml against M8. Intravenous infusion of 400 µg/kg SakSTAR produced 68 ± 18 per cent lysis. These rabbits were then immunized subcutaneously with 400 µg SakSTAR suspended in complete Freund's adjuvant at week 2 and with the same amount in incomplete Freund's adjuvant at weeks 3 and 5. At week 6 the plasma neutralizing activity was increased to 59 ± 47 µg/ml against SakSTAR and to 22 ± 16 µg/ml against M8 whereas the residual thrombolytic potency of 400 µg/kg SakSTAR had decreased to 7.5 ± 2.4 per cent and of 1,000 µg/kg M8 to 4.1 ± 4.8 per cent.

In 8 rabbits assigned to the mutant M9, the baseline neutralizing activity in plasma was 0.2 ± 0.05 µg/ml against SakSTAR and 0.03 ± 0.05 µg/ml against M9. Intravenous infusion of 400 µg/kg M9 produced 72 ± 11 per cent clot lysis. These rabbits were then immunized with 400 µg M9 suspended in complete Freund's adjuvant at week 2 and with the same amount in incomplete Freund's adjuvant at weeks 3 and 5. At week 6, the plasma neutralizing activity was increased to 8.0 ± 4.6 µg/ml against SakSTAR and to 3.5 ± 2.6 µg/ml against M9. At week 6, infusion of 400 µg/kg SakSTAR in 4 of these rabbits produced 53 ± 11 percent clot lysis, while infusion of 400 µg/kg M9 in the 4 other rabbits produced 40 ± 7.8 percent lysis. In 4 control rabbits assigned to SakSTAR, the baseline neutralizing activity in plasma was 0.1 ± 0.05 µg/ml against SakSTAR and 0.05 ± 0.06 µg/ml against M9. Intravenous infusion of 400 µg/kg SakSTAR yielded 78 ± 13 per cent lysis. These rabbits were then immunized with 400 µg SakSTAR suspended in complete (week 2) and incomplete (weeks 3 and 5) Freund's adjuvant respectively. At week 6 the plasma neutralizing activity was increased to 16 ± 5.0 µg/ml against SakSTAR and to 12 ± 9.1 µg/ml against M9 whereas the thrombolytic potency of M9 had decreased to 24 ± 33 per cent.

The immunogenicity of SakSTAR versus the double mutants M3.8 and M8.9 is compared in Table 3B. In 8 rabbits assigned to the M3.8 group, the baseline neutralizing activity in plasma was 0.6 ± 0.3 µg/ml against SakSTAR and 3.5 ± 2.0 µg/ml against M3.8. Intravenous infusion of 1000 µg/kg M3.8 produced 53 ± 13 per cent lysis. These rabbits were then immunized with 400 µg M3.8 suspended in complete Freund's adjuvant at week 2 and with the same amount in incomplete Freund's adjuvant at weeks 3 and 5. At week 6 the plasma neutralizing activity was only increased to 1.7 ± 0.7 µg/ml against SakSTAR and to 6.1 ± 3.0 µg/ml against M3.8. Infusion of 400 µg/kg SakSTAR in 4 of these rabbits produced 77 ± 18 per cent clot lysis whereas infusion of 1000 µg/kg M3.8 in the 4 other rabbits produced 59 ± 25 per cent lysis. In 8 rabbits assigned to the SakSTAR group, the baseline neutralizing activity in plasma was 0.6 ± 0.4 µg/ml against SakSTAR and 2.0 ± 2.0 µg/ml against M3.8. Intravenous infusion of 400 µg/kg SakSTAR produced 80 ± 10 per cent lysis. These rabbits were then immunized subcutaneously with 400 µg SakSTAR suspended in complete Freund's adjuvant at week 2 and with the same amount in incomplete Freund's adjuvant at weeks 3 and 5. At week 6 the plasma neutralizing activity was increased to 20 ± 15 µg/ml against SakSTAR and to 21 ± 22 µg/ml against M3.8 whereas the residual thrombolytic potency of 400 µg/kg SakSTAR had decreased to 8.5 ± 5.7 per cent and of 1,000 µg/kg M3.8 to 30 ± 29 per cent.

In 8 rabbits assigned to the M8.9 group the baseline neutralizing activity in plasma was 0.3 ± 0.2 µg/ml against SakSTAR and 1.6 ± 0.5 µg/ml against M8.9. Intravenous infusion of 800 µg/kg M8.9 produced 39 ± 13 per cent clot lysis at baseline. These 8 rabbits were then immunized with 400 µg M8.9 suspended in complete (week 2) or incomplete (weeks 3 and 5) Freund's adjuvant. At 6 weeks the plasma neutralizing activity was only increased to 2.5 ± 1.5 µg/ml against SakSTAR and to 4.9 ± 1.3 µg/ml against M8.9. At week 6, infusion of 400 µg/kg SakSTAR in 4 of these rabbits produced 51 ± 35 percent clot lysis while infusion of 800 µg/kg M8.9 in the 4 other rabbits produced 39 ± 12 per cent lysis. In 4 control rabbits assigned to SakSTAR the pretreatment neutralizing activity was 0.2 ± 0.1 µg/ml against SakSTAR and 0.7 ± 0.3 µg/l against M8.9. Intravenous infusion of 400 µg/kg SakSTAR induced 67 ± 19 per cent clot lysis. These 4 rabbits were then immunized with 400 µg SakSTAR suspended in complete (week 2) or incomplete (weeks 3 and 5) Freund's adjuvant. At week 6 the plasma neutralizing activity was increased to 20 ± 15 µg/ml against SakSTAR and to 18 ± 15 µg/ml against M8.9 whereas the residual thrombolytic efficacy of M8.9 had only decreased to 31 ± 30 per cent lysis.

These results show that in this directly comparative study of SakSTAR and selected variants, especially the double mutants (M3.8 and M8.9) induce significantly less antibody-related neutralizing activity and resistance to lysis than SakSTAR.

### EXAMPLE 5

### Comparative immunogenicity of SakSTAR and M3.8 in baboons

The comparative immunogenicity of SakSTAR and M3.8 in terms of induction of neutralizing antibodies and refractoriness to thrombolysis on repeated administration was studied in baboons.

The animal experiments were performed according to the guiding principles of the American Physiological Society and the International Committee on Thrombosis and Haemostasis (45). In anesthetized and intubated baboons, an extracorporeal arteriovenous circuit was created by connecting, via an external polyethylene loop, a catheterized tibial or brachial artery to a peripheral vein. A peristaltic pump directed and maintained the continuously monitored blood flow through the extracorporeal loop, in which two adapted hypodermic syringes, each containing one fresh 0.3 ml ¹²⁵I fibrin-labeled pooled baboon plasma clot, were inserted. The time course of clot lysis during infusion of SakSTAR or variant M3.8, was monitored continously by external gamma counting over the syringes. Alternatively, an isotope recovery balance was determined by comparing the sum of the total blood radioactivity count at the end of the experiment (multiplied with a factor 3 to correct for extravascular distribution) plus the radioactivity in the recovered thrombi, with that originally present in the clots.

Before each thrombolysis experiment, baboons were premedicated with an intravenous bolus of ridogrel, 3 mg/kg, to prevent platelet deposition in the extracorporeal system. Throughout the thrombolysis experiments, intravenous heparin was given, as a 300 IU/kg bolus followed by a 200 IU/kg.h infusion.

Twelve adult male baboons (Papio hamadryas) were randomly allocated at baseline (week 0) to treatment with 50 µg/kg of either SakSTAR (group 1) or variant M3.8 (group 2), infused intravenously over one hour with a 10% bolus, and the baseline thrombolytic potency was assessed by monitoring the disappearance of radioactivity from the clots for 2 hours. The baboons were then immunized subcutaneously with 500 µg of either SakSTAR (group 1) or variant M3.8 (group 2), suspended in complete Freund's adjuvant at 2 weeks and in incomplete Freund's adjuvant at 3 and 5 weeks. At 6 weeks, thrombolytic efficacy was quantitated, by means of the extracorporeal thrombolysis model, during 4 hours: 3 of the 6 baboons of group 1, treated at baseline and subsequently immunized with SakSTAR, and 3 of the 6 baboons of group 2, treated at baseline and subsequently immunized with M3.8, were randomly selected and given first 50 µg/kg SakSTAR, infused intravenously over one hour with a 10% bolus and then, after 2 hours from the start of SakSTAR-infusion, the same regimen of M3.8 (groups 1A and 2A, respectively). The other 6 baboons received the same therapy but in reversed order: first M3.8, then SakSTAR (groups 1B and 2B for baboons previously immunized with SakSTAR and M3.8, respectively). At 18 weeks, thrombolytic efficacy was evaluated a third time by monitoring disappearance of radioactivity from fibrin clots, during 3 hours: 3 of the 6 baboons immunized with SakSTAR were given 250 µg/kg SakSTAR as an intravenous bolus over 2.5 min (group 1A) while the 3 other baboons immunized with SakSTAR received the same amount of M3.8 (group 1B). Of the 6 baboons immunized with M3.8, 3 received an intravenous bolus of 250 µg/kg SakSTAR (group 2A) and 3 the same amount of M3.8 (group 2B).

Blood samples were collected on citrated tubes (final concentration 0.01 M) at baseline, and at several time points thereafter for measurement of activated partial thromboplastin time (aPTT), fibrinogen, α₂-antiplasmin (at the start and end of each experiment) and SakSTAR- and M3.8-neutralizing activities (before thrombolytic infusion). Therefore, increasing amounts of either SakSTAR or M3.8 (50 µl volumes containing 0.2 to 1,000 µg/ml), were added to a mixture of 300 µl citrated human plasma and 50 µl buffer or test baboon plasma, immediately followed by addition of 100 µl of a mixture of thrombin (50 NIH U/ml) and CaCl₂ (25 mM). The plasma clot lysis time was measured and plotted against the concentration of SakSTAR or M3.8. From this curve the concentration of plasminogen activator that produced complete clot lysis in 20 min was determined. The neutralizing activity was defined as the difference between the test plasma and buffer values and was expressed in µg/ml plasma.

Systemic fibrinogen was not degraded, nor α₂-antiplasmin depleted, reflecting total fibrin-specificity of both agents.

From 6 weeks on, SakSTAR-neutralizing activities of group 1 were significantly higher than M3.8-neutralizing activities of group 2. Group 1 developed neutralizing activities more rapidly and significantly more markedly against SakSTAR than against M3.8, whereas M3.8-neutralizing activities never surpassed SakSTAR-neutralizing activities in group 2 (Table 4). From 8 weeks on group 1 developed significantly more neutralizing activities both against SakSTAR and against M3.8 than group 2 (table 4).

At baseline, 50 µg/kg SakSTAR infused intravenously over 1 hour, induced 77 ± 2.9% clot lysis over 2 hours in 6 baboons (group 1) and 50 µg/kg M3.8 induced 83 ± 3.6% clot lysis over 2 hours in 6 other baboons (group 2) (mean ± SEM, p= 0.2). At 6 weeks, the lytic efficacy over 2 hours of 50 µg/kg SakSTAR, infused intravenously over 1 hour, declined to 9.2 ± 1.0% in 3 baboons immunized with SakSTAR (group 1A) and to 8.5 ± 3.2% in 3 baboons immunized with M3.8 (group 2A), while the lytic efficacy of 50 µg/kg intravenous M3.8 over 2 hours decreased to 10 ± 6.9% in 3 baboons immunized with SakSTAR (group 1B) and to 11 ± 7.4% in 3 baboons immunized with M3.8 (group 2B; p < 0.001 versus corresponding baseline lysis for all groups; p= NS between groups). Two hours after the start of the first thrombolytic infusion, 50 µg/kg of the other agent was infused intravenously over 1 hour, yielding comparable residual lytic efficacies: 7.7 ± 1.5% and 11 ± 5.7% clot lysis over 2 hours with M3.8 in groups 1A and 2A, respectively, and 13 ± 2.7% and 12 ± 2.1% clot lysis over 2 hours with SakSTAR in groups 1B and 2B, respectively.

At 18 weeks, intravenous bolus injection of 250 µg/kg SakSTAR yielded 39 ± 5.3% clot lysis over 3 hours in 3 baboons immunized with SakSTAR (group 1A), whereas the residual thrombolytic potency of 250 µg/kg M3.8 in 3 baboons immunized with M3.8 (group 2B) was significantly greater: 68 ± 4.5% clot lysis over 3 hours (p< 0.005). 250 µg/kg SakSTAR produced 58 ± 9.5% clot lysis over 3 hours in 3 baboons immunized with M3.8 (group 2A; p= 0.1 vs group 1A), while clot lysis over 3 hours with 250 µg/kg M3.8 in 3 baboons immunized with SakSTAR (group 1B) was 39 ± 3.6% (p= 0.0005 vs group 2B). Pooled analysis, irrespective of the agent administered at 18 weeks, showed a residual lysis over 3 hours of 39 ± 3.0%, for group 1, immunized with SakSTAR, versus 63 ± 5.2% for group 2, immunized with M3.8 (p < 0.0005).

Thrombolytic potencies correlated inversely with the corresponding neutralizing activities throughout the study period (Spearman r= -0.83; p < 0.0001).

Thus, the mutant M3.8 is comparably active and fibrin-specific but significantly less antigenic than wild-type SakSTAR in baboons, as evidenced by less induction of neutralizing activities in plasma and by faster recovery of thrombolytic potential after immunization. These results, obtained in outbred primates, confirm and extend the above observations in rabbits.

### EXAMPLE 6

### Comparative thrombolytic efficacy and immunogenicity of M3.8 and M8.9 versus SakSTAR in patients with peripheral arterial occlusion

SakSTAR (n= 8), M3.8 (n= 4) and M8.9 (n= 4) were administered intra-arterially at or in the proximal end of the occlusive thrombus as a bolus of 2 mg followed by an infusion of 1 mg per hr in patients with angiographically documented arterial occlusion of a peripheral artery or bypass graft. Patients were studied after giving informed consent, and the protocol was approved by the Human Studies Committee of the University of Leuven. Inclusion and exclusion criteria were essentially as previously described (46) except that retreatment with recombinant staphylokinase moiety within 48 hrs was allowed. Conjunctive antithrombotic treatment with heparin, aspirin and oral anticoagulants was as previously described (46).

The patency status of the occluded peripheral artery or bypass graft was serially evaluated before, at least every 4 hours during, and at the end of the intra-arterial infusion of wild type or variant SakSTAR. The angiographic patency status of the target vessel at the end of the infusion constituted the main study endpoint. The administration of thrombolytic agent was terminated when adequate vessel patency was achieved, when complications required its cessation or when two consecutive angiograms failed to demonstrate progression of clot lysis. Recanalization was defined as clot lysis sufficient to restore brisk anterograde flow throughout the previously occluded segment. Complementary intravascular procedures such as percutaneous transluminal angioplasty (PTA) were allowed when the investigators judged that the thrombus was sufficiently lysed or that no further thrombolysis was to be expected.

Blood pressure and heart rate were monitored before, during and after infusion of SakSTAR, M3.8 or M8.9. Blood samples were collected before, at the end of, and 6 hours after the angiographic procedure. Measurements included peripheral blood count, prothrombin time (PT), aPTT, fibrinogen, α₂-antiplasmin, plasminogen, and biochemical hepatic and renal function tests. SakSTAR-neutralizing, M3.8-neutralizing and M8.9-neutralizing activities and anti-SakSTAR, anti-M3.8 and anti-M8.9 IgG and IgM were serially determined on blood samples drawn during hospitalization and after discharge. Clinical follow-up focussed on recurrence of thrombosis and on adverse events such as allergic reactions and major bleeding (i.e. need for blood transfusion or surgical control, drop of hematocrit of > 10%, or intracranial bleeding).

Groups of 4 to 8 patients (41 to 73 years) with angiographically documented PAO, with an estimated duration of 1 to 120 days and a length of 8 to 50 cm, were treated with M3.8, M8.9 or SakSTAR. One patient (WAL) given wild-type SakSTAR developed an anaphylactoid reaction within 5 min after the 2 mg bolus administration. The infusion was immediately interrupted and the blood pressure returned to normal within 20 min during infusion of plasma expanders. This patient was not included for calculation of mean ± SEM in Tables 5 to 7. One patient (LAN) given M8.9 developed reocclusion after 30 hrs, which was treated with 6.5 mg of the variant. This patient developed 7.8 µg/ml M8.9 neutralizing activity and 270 µg/ml specific anti-M8.9 IgG after 2-3 weeks.

Relevant baseline characteristics of the individual patients are shown in Table 5. The majority of PAO were at the femoropopliteal level. All were due to in situ thrombosis. Two grafts and 2 iliac stent occlusions were included. Nine patients presented with incapacitating claudication, 2 with chronic ischemic rest pain, 4 with subacute and 1 with acute ischemia.

Table 6 summarizes the individual results of treatment and outcome. Intra-arterial infusion, at a dose of 5.5 to 13 mg during 3.5 to 11 hrs, induced complete recanalization in 13 patients, partial recanalization in 1 patient and no improvement in 1 patient. Complementary endovascular procedures (mainly PTA) were performed in 12 and complementary surgery immediately following thrombolysis in 1 patient. Recurrence of thrombosis after the end of the angiographic procedure occurred in 4 patients: the first patient was successfully retreated after 30 hrs with 6.5 mg M8.9, the second underwent aortabi-iliac bypass grafting, the third patient was successfully recanalized after 20 hrs with 40 mg rt-PA and the thrombus was aspirated transluminally in the fourth patient. Bleeding complications were absent or limited to mild to moderate hematoma formation at the angiographic puncture sites except for one patient who developed a hematoma within the right quadriceps muscle. Other complications related to endovascular manipulations included distal embolization and arterial dissection which necessitated premature cessation of thrombolytic infusion in one patient. One superficial femoral arterial occlusion proved to be resistant to 8.0 mg SakSTAR infused over 6.0 hrs and was subsequently managed successfully by PTA (Table 6).

Circulating fibrinogen, plasminogen and α₂-antiplasmin levels remained unchanged during infusion of the SakSTAR moieties (Table 7), reflecting absolute fibrin specificity of these agents at the dosages used. Substantial in vivo fibrin digestion occurred as evidenced by elevation of D-dimer levels. Intra-arterial heparin therapy prolonged the aPTT (Table 7).

Antibody-related SakSTAR-, M3.8- and M8.9-neutralizing activity and anti-SakSTAR-, M3.8- and M8.9-specific IgG, were low at baseline and during the first week after the infusion (Table 8). From the second week on neutralizing activity levels increased to median values of 2.9 µg and 3.3 µg SakSTAR variant neutralized per ml plasma in the patients treated with M3.8 and M8.9, respectively, which is significantly lower than the median value of 9.1 µg wild-type SakSTAR neutralized per ml in the patients treated with SakSTAR (p= 0.03 for variants vs wild-type by Mann-Whitney rank sum test). The levels of SakSTAR-specific IgG increased to median values of 51 and 31 µg/ml plasma in patients treated with M3.8 and M8.9, respectively, which is significantly lower than the median value of 240 µg/ml in the patients treated with SakSTAR (p= 0.01 for variants vs wild-type by Mann-Whitney rank sum test).

Thus, in patients with peripheral arterial occlusion given doses of 5.5 to 13 mg of compound, M3.8 and M8.9 induced significantly less neutralizing antibodies and specific anti-staphylokinase IgG than SakSTAR. These variants provide proof of concept that reduction of the humoral response against recombinant staphylokinase by protein engineering is feasible.

### LEGENDS TO THE FIGURES

Fig 1. Protein sequence of wild-type staphylokinase, SakSTAR. Numbering starts with the NH₂-terminal amino acid of mature full length staphylokinase. The "charge-cluster to alanine" variants that were studied are indicated.
Fig 2. Schematic representation of the epitope specificity of a panel of 17 murine monoclonal antibodies raised against SakSTAR.

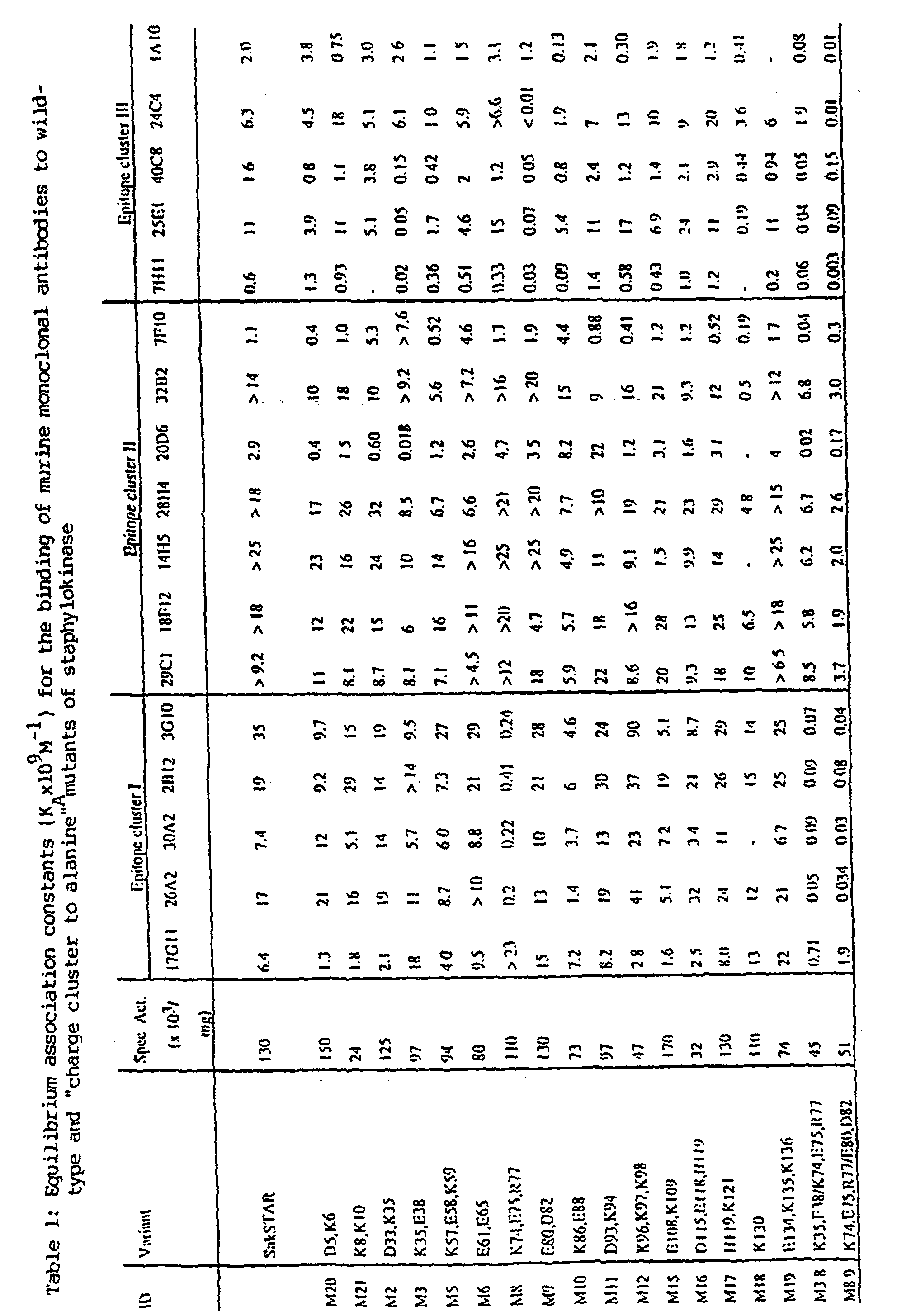

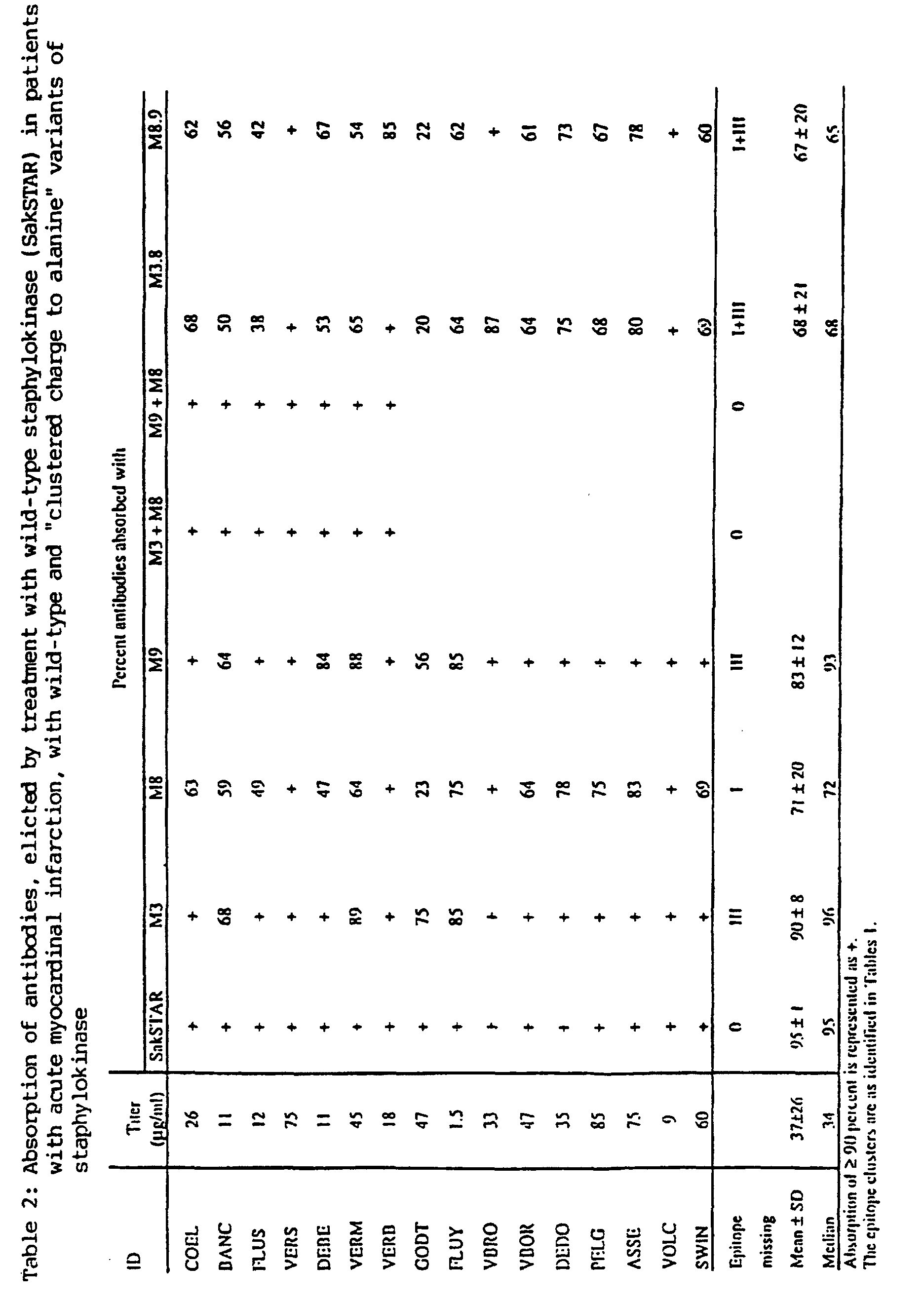

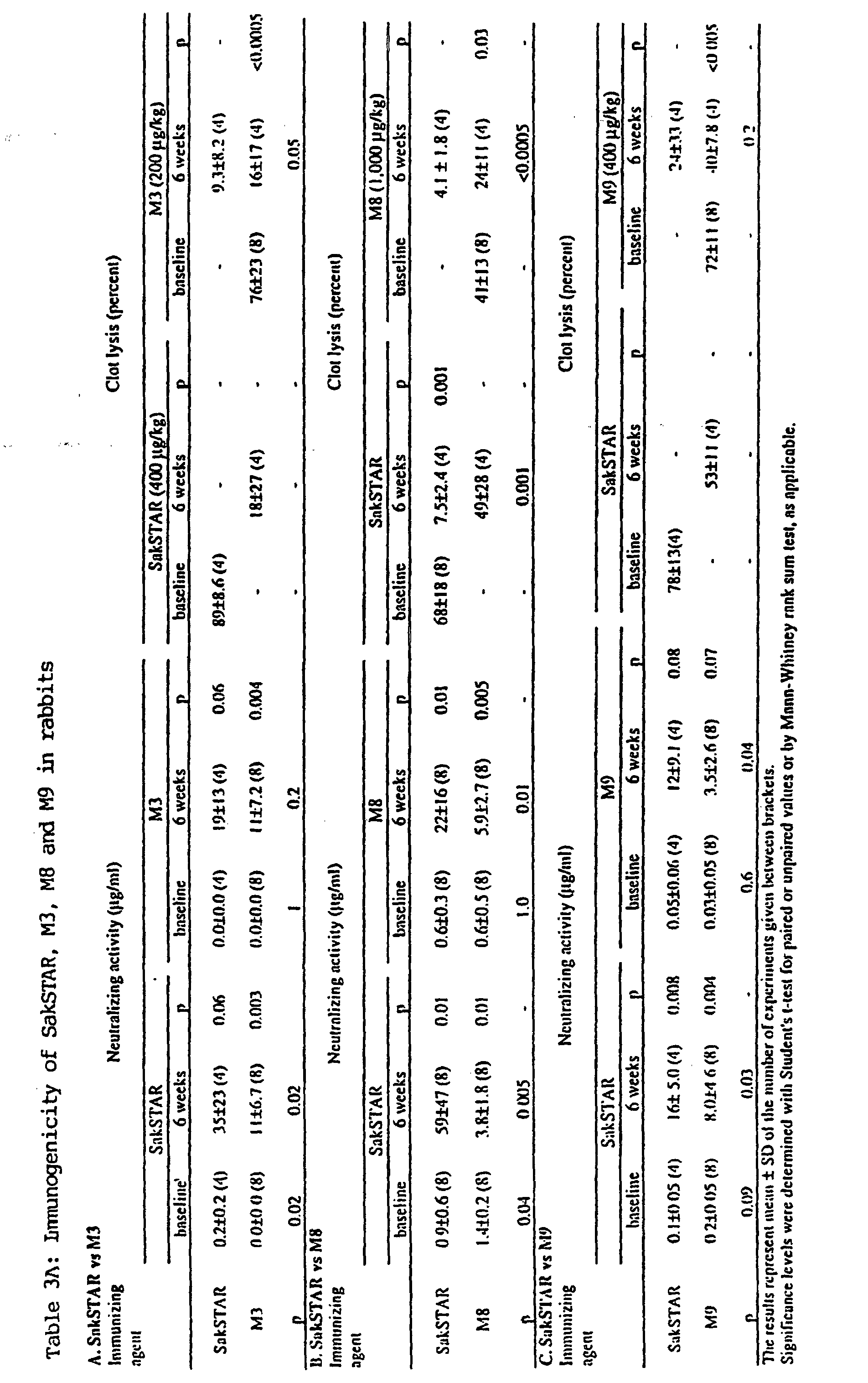

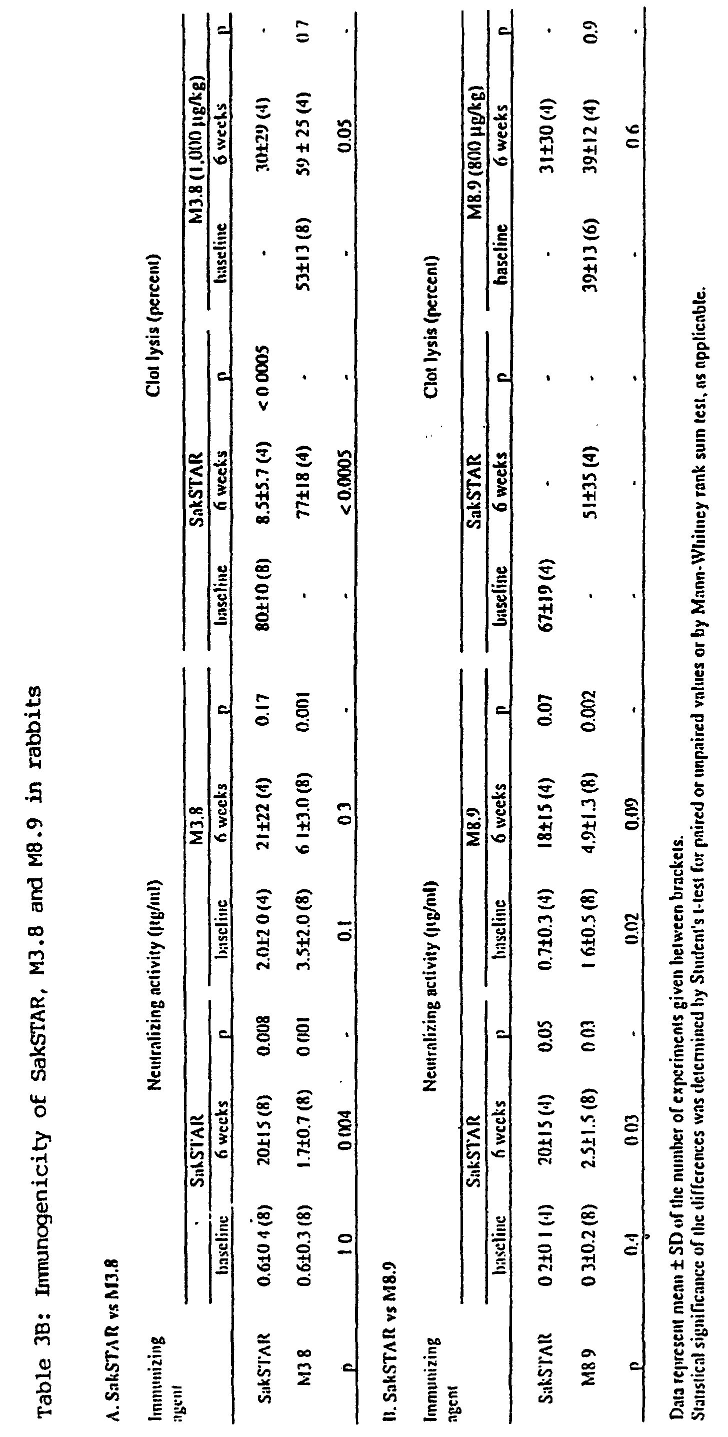

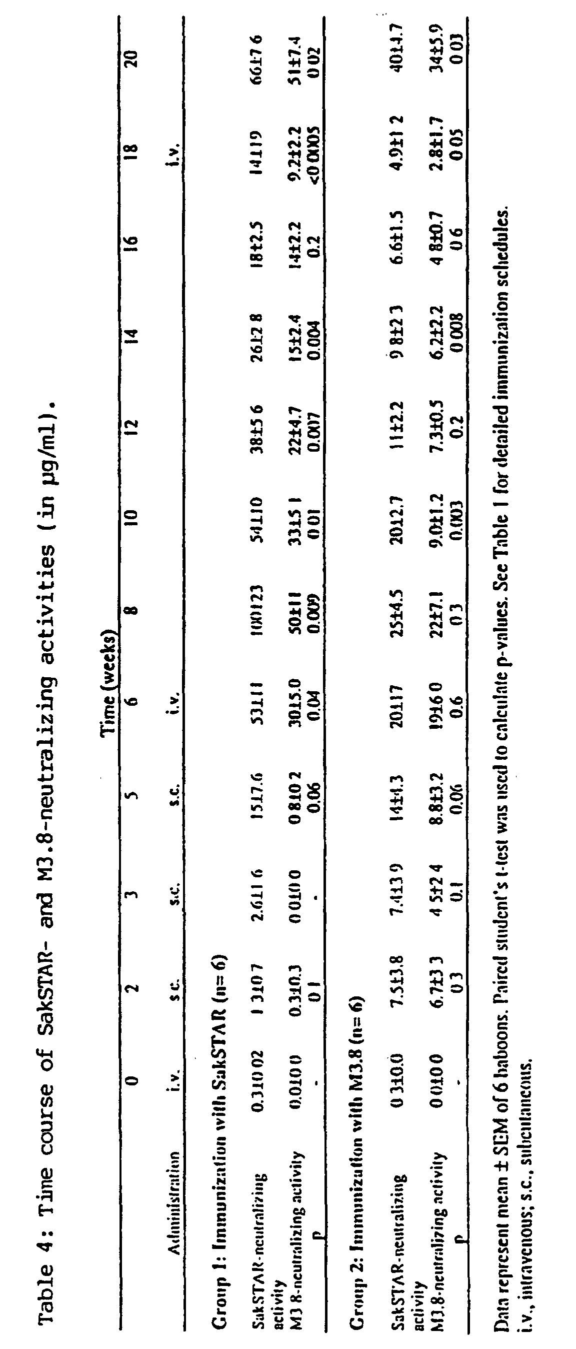

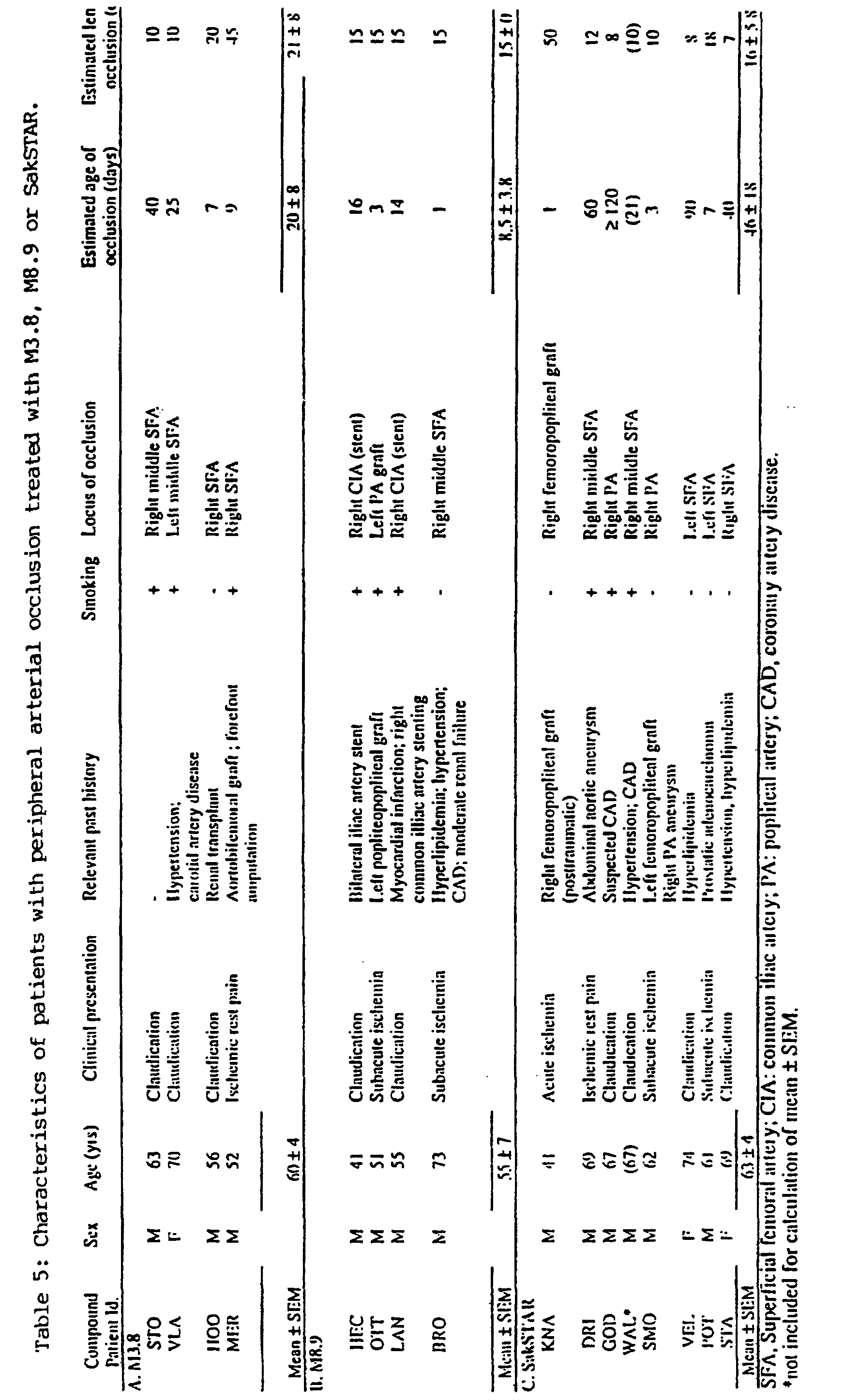

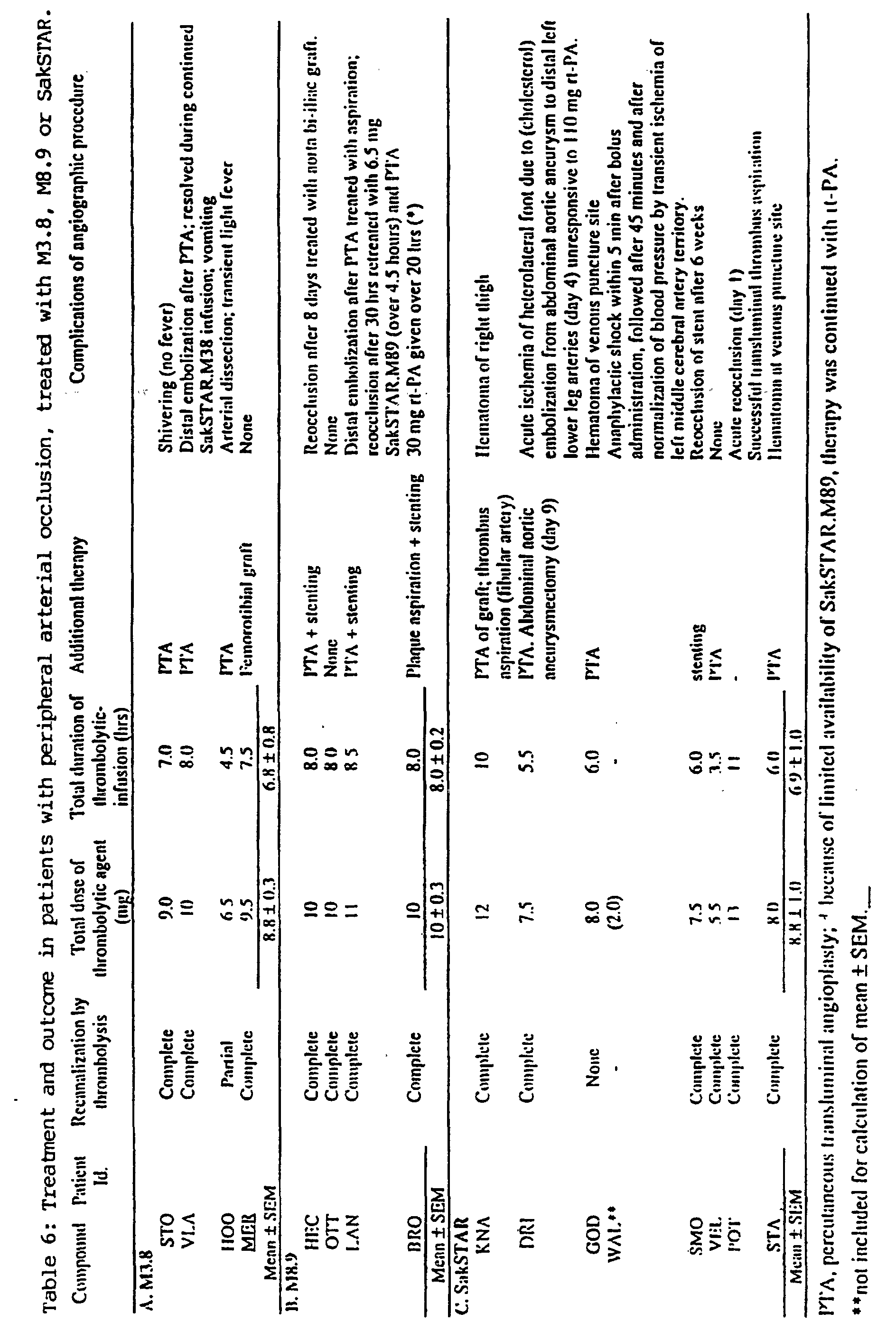

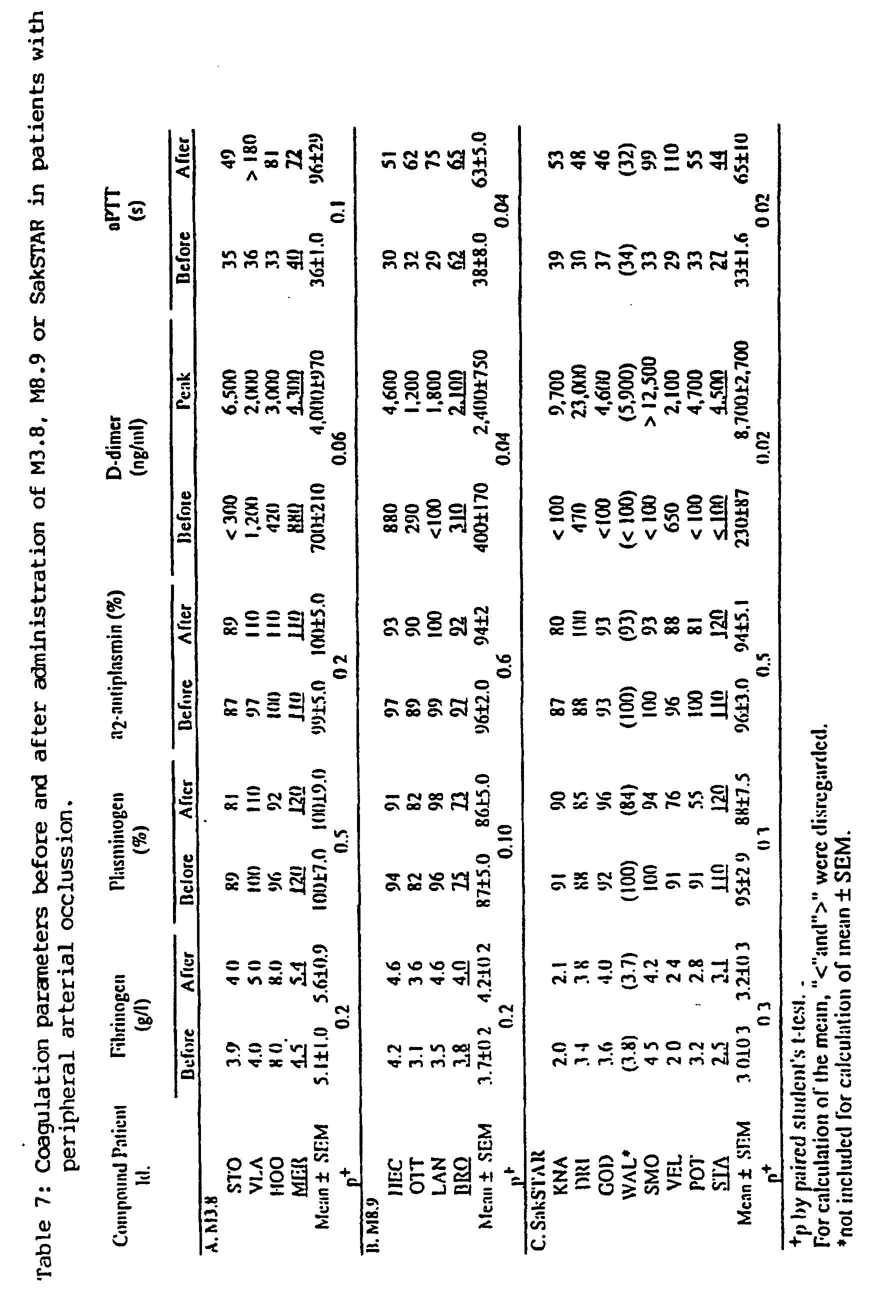

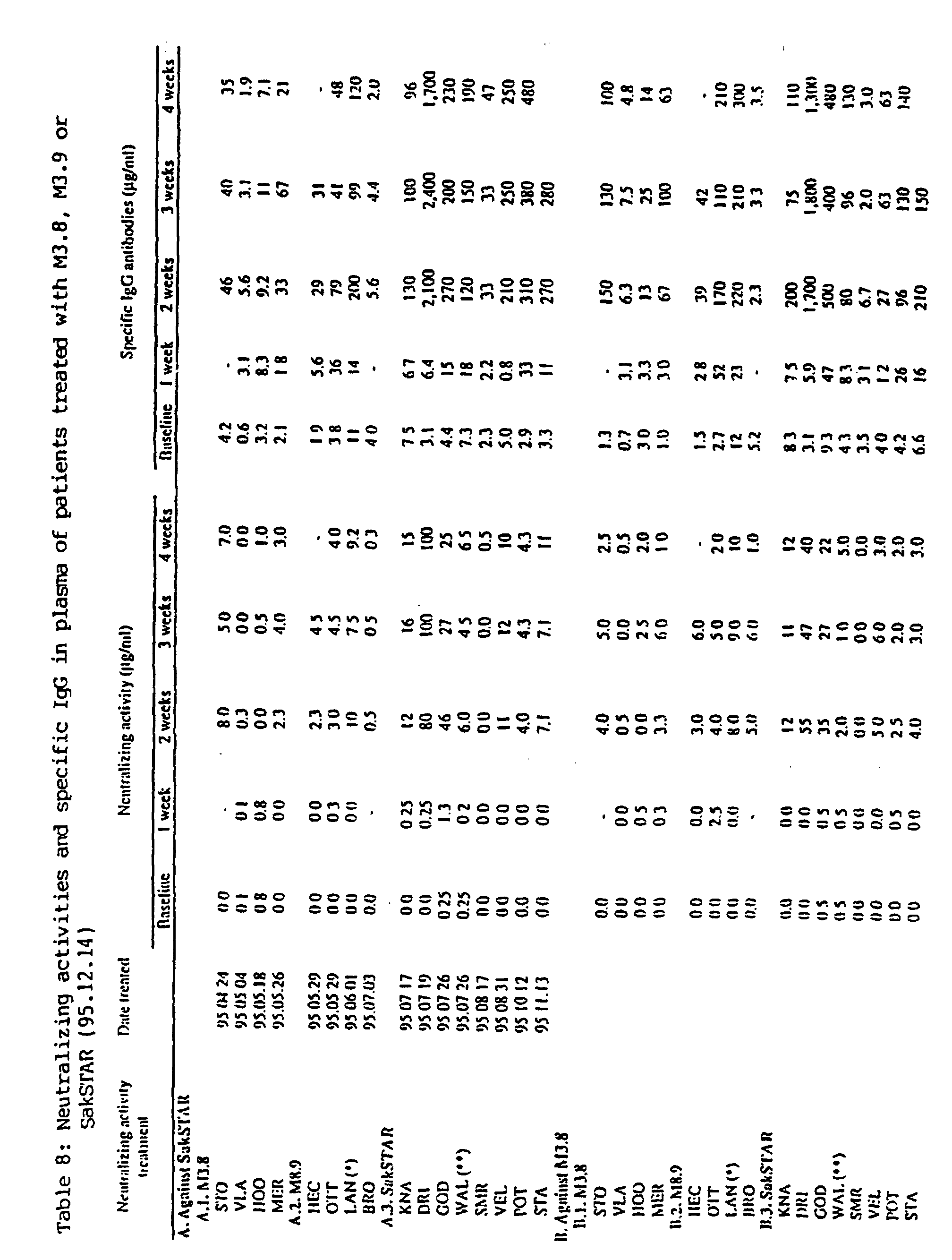

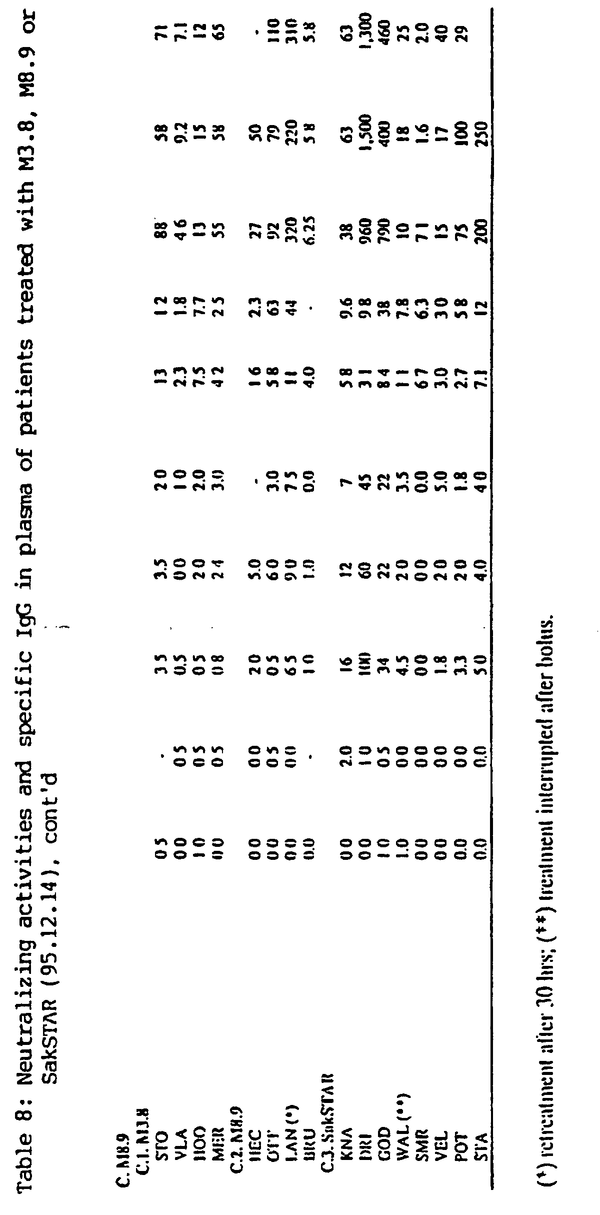

### REFERENCES

1. Collen D: On the regulation and control of fibrinolysis. Edward Kowalski Memorial Lecture. Thromb Haemostas 43: 77-79, 1980.
2. Collen D, Lijnen HR: Basic and clinical aspects of fibrinolysis and thrombolysis. Blood 78: 3114-3124, 1991.
3. Collen D, Van de Werf F: Coronary thrombolysis with recombinant staphylokinase in patients with evolving myocardial infarction. Circulation 87: 1850-1853, 1993.
4. Vanderschueren S, Collen D: Immunogeniciteit van streptokinase en implicaties voor gebruik. Tijdschr Geneesk 50: 1639-1644, 1994.
5. Lack CH: Staphylokinase: an activator of plasma protease. Nature 161: 559, 1948.
6. Lewis JH, Ferguson JH: A proteolytic enzyme system of the blood. III. Activation of dog serum profibrinolysin by staphylokinase. Am J Physiol 166: 594, 1951.
7. Winkler KC, DeWaart J, Grootsen C, Zegers BJM, Tellier NF, Vertegt CD: Lysogenic conversion of staphylococci to loss of beta-toxin. J Gen Microbiol 39: 321, 1965.
8. Collen D, Lijnen HR: Staphylokinase, a fibrin-specific plasminogen activator with therapeutic potential ? Blood 84: 680-686,1994.
9. Sako T, Sawaki S, Sakurai T, Ito S, Yoshizawa Y, Kondo I: Cloning and expression of the staphylokinase gene of Staphylococcus aureus in Escherichia coli. Molec Gen Genet 190: 271-277, 1983.
10. Behnke D, Gerlach D: Cloning and expression in Escherichia coli, Bacillus subtilis, and Streptococcus sanguis of a gene for staphylokinase - a bacterial plasminogen activator. Molec Gen Genet 210: 528-534, 1987.
11. Collen D, Silence K, Demarsin E, De Mol M, Lijnen HR: Isolation and characterization of natural and recombinant staphylokinase. Fibrinolysis 6: 203-213, 1992.
12. Sako T: Overproduction of staphylokinase in Escherichia coli and its characterization. Eur J Biochem 149: 557-563, 1985.
13. Gerlach D, Kraft R, Behnke D: Purification and characterization of the bacterial plasminogen activator staphylokinase secreted by a recombinant bacillus subtilis. Zbl Bakt Mikr Hyg 269: 314,-322 1988.
14. Sako T, Tsuchida N: Nucleotide sequence of the staphylokinase gene from Staphylococcus aureus. Nucleic Acids Res 11: 7679-7693, 1983.
15. Collen D, Zhao ZA, Holvoet P, Marynen P: Primary structure and gene structure of staphylokinase. Fibrinolysis 6: 226-231, 1992.
16. Schlott B, Hartmann M, Gührs KH, Birch-Hirschfeld E, Gase A, Vetterman S, Collen D, Lijnen HR: Functional properties of recombinant staphylokinase variants obtained by site-specific mutagenesis of methionine-26. Biochim Biophys Acta 1204: 235-242, 1994.
17. Sakai M, Watanuki M, Matsuo O: Mechanism of fibrin-specific fibrinolysis by staphylokinase: participation of α₂-plasmin inhibitor. Biochem Biophys Res Comm 162: 830-837, 1989.
18. Matsuo O, Okada K, Fukao H, Tomioka Y, Ueshima S, Watanuki M, Sakai M: Thrombolytic properties of staphylokinase. Blood 76: 925-929, 1990.
19. Lijnen HR, Van Hoef B, De Cock F, Okada K, Ueshima S, Matsuo O, Collen D: On the mechanism of fibrin-specific plasminogen activation by staphylokinase. J Biol Chem 266: 11826-11832, 1991.
20. Lijnen HR, Van Hoef B, Matsuo O, Collen D: On the molecular interactions between plasminogen-staphylokinase, α₂-antiplasmin and fibrin. Biochim Biophys Acta 1118: 144-148, 1992.
21. Silence K, Collen D, Lijnen HR: Interaction between staphylokinase, plasmin(ogen) and α₂-antiplasmin. Recycling of staphylokinase after neutralization of the plasmin-staphylokinase complex by α₂-antiplasmin. J Biol Chem 268: 9811-9816, 1993.
22. Silence K, Collen D, Lijnen HR: Regulation by α₂-antiplasmin and fibrin of the activation of plasminogen with recombinant staphylokinase in plasma. Blood 82: 1175-1183, 1993.
23. Collen D, De Cock F, Vanlinthout I, Declerck PJ, Lijnen HR, Stassen JM: Comparative thrombolytic and immunogenic properties of staphylokinase and streptokinase. Fibrinolysis 6: 232-242, 1992.
24. Collen D, De Cock F, Stassen JM: Comparative immunogenicity and thrombolytic properties toward arterial and venous thrombi of streptokinase and recombinant staphylokinase in baboons. Circulation 87: 996-1006, 1993.
25. Schlott B, Hartmann M, Gührs KH, Birch-Hirschfeid E, Pohl HD, Vanderschueren S, Van de Werf F, Michoel A, Collen D, Behnke D: High yield production and purification of recombinant staphylokinase for thrombolytic therapy. Bio/technology 12: 185-189, 1994.
26. Declerck PJ, Vanderschueren S, Billiet J, Moreau H, Collen D: Prevalence and induction of circulating antibodies against recombinant staphylokinase. Thromb Haemostas 71: 129-133, 1994.
27. Vanderschueren SMF, Stassen JM, Collen D: On the immunogenicity of recombinant staphylokinase in patients and in animal models. Thromb Haemostas 72: 297-301, 1994.
28. White H: Thrombolytic treatment for recurrent myocardial infarction. Br Med J 302: 429-430, 1991.
29. Gase A, Hartmann M, Gührs KH, Röcker A, Collen D, Behnke D, Schlott B: Functional significance of NH₂-and COOH-terminal regions of staphylokinase in plasminogen activation. Submitted.
30. Galfré G, Milstein C: Preparation of monoclonal antibodies: strategies and procedures. Methods Enzymol 73: 3-46, 1981.
31. de St. Groth SF, Scheidegger D: Production of monoclonal antibodies: strategies and tactics. J Immunol Methods 35: 1-21, 1980.
32. Nakane PK, Kawaoi A: Peroxidase-labeled antibody. A new method for conjugation. J Histochem Cytochem 22: 1084-1091, 1974.
33. Anderson N, Potter M: Induction of plasma cell tumours in Balb-c mice with 2, 6, 10, 14 tetramethylpentadecane (pristane). Nature 222: 994-995, 1969.
34. Ey PL, Prowse SJ, Jenkin CR: Isolation of pure IgG₁, IgG₂ₐ and IgG_{2b} immunoglobulins from mouse serum using protein A-Sepharose. Immunochemistry 15: 429-436, 1978.
35. Jönsson U, Malmqvist M: Real time biospecific interaction analysis. The integration of surface plasmon resonance detection, general biospecific interface chemistry and microfluidics into one analytical system. Adv Biosensors 2: 291-336, 1992.
36. Johnsson B, Löfas S, Lindquist G: Immobilization of proteins to a carboxymethyldextran-modified gold surface for biospecific interaction analysis in surface plasmon resonance sensors. Anal Biochem 198: 268-277, 1991.
37. BIAcore system manual, 5-2, Pharmacia Biosensor AB, Uppsala, Sweden.
38. Karlsson R, Michaelsson A, Mattsson L: Kinetic analysis of monoclonal antibody-antigen interactions with a new biosensor based analytical system. J Immunol Methods 145: 229-240, 1991.
39. Stanssens P, Opsomer C, McKeown Y, Kramer W, Zabeau M, Friz MJ: Efficient oligonucleotide-directed construction of mutations in expression vectors by the gapped duplex DNA method using alternating selectable markers. Nucleic Acids Res 17: 4441-4454, 1989.
40. Deutsch DG, Mertz ET: Plasminogen: purification from human plasma by affinity chromatography. Science 170: 1095-1096, 1970.
41. Silence K, Hartmann M, Gührs KH, Gase A, Schlott B, Collen D, Lijnen HR: Structure-function relationships in staphylokinase as revealed by "clustered-charge-to-alanine" mutagenesis. J Biol Chem (in press).
42. Bradford MM: A rapid and sensitive method for the quantitation of microgram quantities of protein utilizing the principle of protein-dye binding. Anal Biochem 72: 248, 1976.
43. De Clerck F, Beetens J, de Chaffoy de Courcelles D, Freyne E, Janssen PA: R68070: thromboxane A2 synthetase inhibition and thromboxane A2/prostaglandin endoperoxide receptor blockade combined in one molecule. I. Biochemical profile in vitro. Thromb Haemost 61: 35-42, 1989.
44. Stassen JM, Vanlinthout I, Lijnen HR, Collen D: A hamster pulmonary embolism model for the evaluation of the thrombolytic and pharmacokinetic properties of thrombolytic agents. Fibrinolysis 4 (Suppl 2): 15-21, 1990.
45. Giles AR: Guidelines for the use of animals in biomedical research. Thromb Haemost 58: 1078-1084, 1987.
46. Vanderschueren S, Stockx L, Wilms G, Lacroix H, Verhaeghe R, Vermylen J, Collen D: Thrombolytic therapy of peripheral arterial occlusion with recombinant staphylokinase. Circulation 92: 2050-2057, 1995.
47. Vanderschueren S, Barrios L, Kerdsinchai P, Van den Heuvel P, Hermans L, Vrolix M, De Man F, Benit E, Muyldermans L, Collen D, Van de Werf F: A randomized trial of recombinant staphylokinase versus alteplase for coronary artery patency in acute myocardial infarction. Circulation 92: 2044-2049, 1995.

## Claims

1. Staphylokinase derivatives showing a reduced immunogenicity as compared to wild-type staphylokinase having essentially the amino acid sequence as depicted in figure 1 in which one or more amino acids in one or more of the underlined clusters have been replaced by another amino acid thus destroying the corresponding epitope(s).

2. Staphylokinase derivatives as claimed in claim 1 having essentially the amino acid sequence as depicted in figure 1 in which one or more amino acids in one or more of the underlined clusters have been replaced by alanine thus destroying the corresponding epitope(s).

3. Staphylokinase derivative M8 having the amino acid sequence as depicted in figure 1 in which the amino acids Lys on position 74, Glu on position 75 and Arg on position 77 in the underlined cluster 8 have been replaced by alanine thus altering the corresponding epitope.

4. Staphylokinase derivative M3 having the amino acid sequence as depicted in figure 1 in which the amino acids Lys on position 35 and Glu on position 38 in the underlined cluster 3 have been replaced by alanine thus altering the corresponding epitope.

5. Staphylokinase derivative M9 having the amino acid sequence as depicted in figure 1 in which the amino acids Glu on position 80 and Asp on position 82 in the underlined cluster 9 have been replaced by alanine thus altering the corresponding epitope.

6. Staphylokinase derivative M3.8 having the amino acid sequence as depicted in figure 1 in which the amino acids Lys on position 35, Glu on position 38, Lys on position 74, Glu on position 75 and Arg on position 77 in the underlined clusters 3 and 8 have been replaced by alanine thus altering the corresponding epitope.

7. Staphylokinase derivative M8.9 having the amino acid sequence as depicted in figure 1 in which the amino acids Lys on position 74, Glu on position 75, Arg on position 77, Glu on position 80 and Asp on position 82 in the underlined clusters 8 and 9 have been replaced by alanine, thus altering the corresponding epitope.

8. Pharmaceutical composition comprising at least one of the staphylokinase derivatives as claimed in any one of the claims 1-7 together with a suitable excipient.

9. Pharmaceutical composition as claimed in claim 8 for treating arterial thrombosis.

10. Staphylokinase derivatives as claimed in any one of the claims 1-7 for use of the treatment of arterial thrombosis.

11. Use of staphylokinase derivatives as claimed in any one of the claims 1-7 for the preparation of a pharmaceutical composition for the treatment of arterial thrombosis.

## Patentansprüche

1. Staphylokinase-Derivate, die eine reduzierte Immunogenität im Vergleich zur Wildtyp-Staphylokinase zeigen, mit im Wesentlichen der Aminosäuresequenz, wie sie in Figur 1 dargestellt ist, bei welcher eine oder mehrere Aminosäuren in einem oder mehreren der unterstrichenen Cluster durch eine andere Aminosäure ersetzt worden ist/sind, sodass das/die entsprechende(n) Epitop(e) zerstört ist/sind.

2. Staphylokinase-Derivate nach Anspruch 1,
welche im Wesentlichen die Aminosäuresequenz aufweisen, wie sie in Figur 1 dargestellt ist, bei welcher eine oder mehrere Aminosäuren in einem oder mehreren der unterstrichenen Cluster durch Alanin ersetzt worden ist/sind, sodass das/die entsprechende(n) Epitop(e) zerstört ist/sind.

3. Staphylokinase-Derivat M8, welches die Aminosäuresequenz aufweist, wie sie in Figur 1 dargestellt ist, bei welcher die Aminosäuren Lys auf Position 74, Glu auf Position 75 und Arg auf Position 77 in dem unterstrichenen Cluster 8 durch Alanin ersetzt worden sind, sodass das entsprechende Epitop geändert ist.

4. Staphylokinase-Derivat M3, welches die Aminosäuresequenz aufweist, wie sie in Figur 1 dargestellt ist, bei welcher die Aminosäuren Lys auf Position 35 und G!u auf Position 38 in dem unterstrichenen Cluster 3 durch Alanin ersetzt worden sind, sodass das entsprechende Epitop geändert ist.

5. Staphylokinase-Derivat M9, welches die Aminosäuresequenz aufweist, wie sie in Figur 1 dargestellt ist, bei welcher die Aminosäuren Glu auf Position 80 und Asp auf Position 82 in dem unterstrichenen Cluster 9 durch Alanin ersetzt worden sind, sodass das entsprechende Epitop geändert ist.

6. Staphylokinase-Derivat M3.8, welches die Aminosäuresequenz aufweist, wie sie in Figur 1 dargestellt ist, bei welcher die Aminosäuren Lys auf Position 35, Glu auf Position 38, Lys auf Position 74, Glu auf Position 75 und Arg auf Position 77 in den unterstrichenen Clustern 3 und 8 durch Alanin ersetzt worden sind, sodass das entsprechende Epitop geändert ist.

7. Staphylokinase-Derivat M8.9, welches die Aminosäuresequenz aufweist, wie sie in Figur 1 dargestellt ist, bei welcher die Aminosäuren Lys auf Position 74, Glu auf Position 75, Arg auf Position 77, Glu auf Position 80 und Asp auf Position 82 in den unterstrichenen Clustern 8 und 9 durch Alanin ersetzt worden sind, sodass das entsprechende Epitop geändert ist.

8. Pharmazeutische Zusammensetzung, welche zumindest eines der Staphylokinase-Derivate gemäß einem der Ansprüche 1 bis 7 zusammen mit einem geeigneten Exzipienten umfasst.

9. Pharmazeutische Zusammensetzung nach Anspruch 8 zur Behandlung von arterieller Thrombose.

10. Staphylokinase-Derivate gemäß einem der Ansprüche 1 bis 7 zur Verwendung für die Behandlung von arterieller Thrombose.

11. Verwendung von Staphylokinase-Derivaten gemäß einem der Ansprüche 1 bis 7 für die Darstellung einer pharmazeutischen Zusammensetzung zur Behandlung von arterieller Thrombose.

## Revendications

1. Dérivés de staphylokinase manifestant une immunogénicité réduite comparativement à la staphylokinase de type sauvage ayant essentiellement la séquence d'acides aminés illustrée sur la figure 1 dans laquelle un ou plusieurs acides aminés dans un ou plusieurs des ensembles soulignés ont été remplacés par un autre acide aminé, en détruisant ainsi l'épitope ou les épitopes correspondants.

2. Dérivés de staphylokinase selon la revendication 1 ayant essentiellement la séquence d'acides aminés illustrée sur la figure 1 dans laquelle un ou plusieurs acides aminés dans un ou plusieurs des ensembles soulignés ont été remplacés par une alanine, en détruisant ainsi l'épitope ou les épitopes correspondants.

3. Dérivé de staphylokinase M8 ayant la séquence d'acides aminés illustrée sur la figure 1 dans laquelle les acides aminés Lys à la position 74, Glu à la position 75 et Arg à la position 77 dans l'ensemble 8 souligné ont été remplacés par une alanine, en modifiant ainsi l'épitope correspondant.

4. Dérivé de staphylokinase M3 ayant la séquence d'acides aminés illustrée sur la figure 1 dans laquelle les acides aminés Lys à la position 35 et Glu à la position 38 dans l'ensemble 3 souligné ont été remplacés par une alanine, en modifiant ainsi l'épitope correspondant.

5. Dérivé de staphylokinase M9 ayant la séquence d'acides aminés illustrée sur la figure 1 dans laquelle les acides aminés Glu à la position 80 et Asp à la position 82 dans l'ensemble 9 souligné ont été remplacés par une alanine, en modifiant ainsi l'épitope correspondant.

6. Dérivé de staphylokinase M3.8 ayant la séquence d'acides aminés illustrée sur la figure 1 dans laquelle les acides aminés Lys à la position 35, Glu à la position 38, Lys à la position 74, Glu à la position 75 et Arg à la position 77 dans les ensembles 3 et 8 soulignés ont été remplacés par une alanine, en modifiant ainsi l'épitope correspondant.

7. Dérivé de staphylokinase M8.9 ayant la séquence d'acides aminés illustrée sur la figure 1 dans laquelle les acides aminés Lys à la position 74, Glu à la position 75, Arg à la position 77, Glu à la position 80 et Asp à la position 82 dans les ensembles 8 et 9 soulignés ont été remplacés par une alanine, en modifiant ainsi l'épitope correspondant.

8. Composition pharmaceutique comprenant au moins un des dérivés de staphylokinase selon l'une quelconque des revendications 1-7 conjointement avec un excipient convenable.

9. Composition pharmaceutique selon la revendication 8 pour traiter la thrombose artérielle.

10. Dérivés de staphylokinase selon l'une quelconque des revendications 1-7 utilisables pour le traitement de la thrombose artérielle.

11. Utilisation des dérivés de staphylokinase selon l'une quelconque des revendications 1-7 pour la préparation d'une composition pharmaceutique pour le traitement de la thrombose artérielle.
